# EUROPÄISCHE PATENTSCHRIFT

(11) **EP 3 646 861 B1**
(45) Veröffentlichungstag und Bekanntmachung des Hinweises auf die Patenterteilung: **16.08.2023**
(21) Anmeldenummer: 18204420.6
(22) Anmeldetag: 05.11.2018
(51) Int. Cl.: A61K 31/21, A61K 31/522, A61K 36/185, A61K 36/31, A61P 9/12

(54) **ZUSAMMENSETZUNG MIT NITRAT, KOFFEIN UND SULFORAPHAN ZUR BLUTDRUCKSENKUNG**
BLOOD PRESSURE LOWERING COMPOSITION WITH NITRATES, CAFFEINE AND SULFORAPHANE
COMPOSITION HYPOTENSIVE COMPRENANT DES NITRATES, CAFFÉINE ET SULFORAPHANE

(43) Veröffentlichungstag der Anmeldung: 06.05.2020
(73) Patentinhaber: Kruse, Marcus, 45130 Essen (DE); Rassaf, Tienush, 45133 Essen (DE)
(72) Erfinder: Kruse, Marcus, 45130 Essen (DE); Rassaf, Tienush, 45133 Essen (DE)
(74) Vertreter: Von Rohr Patentanwälte Partnerschaft mbB

(56) Entgegenhaltungen:
- JP-A- 2004 083 529
- US-A1- 2011 189 319
- US-A1- 2013 136 810
- US-A1- 2018 133 247
- OSKARSSON JOHANNA ET AL: "No individual or combined effects of caffeine and beetroot-juice supplementation during submaximal or maximal running", APPLIED PHYSIOLOGY, NUTRITION, AND METABOLISM = PHYSIOLOGIE APPLIQUEE, NUTRITION ET METABOLISME FEB 2017,, Bd. 43, Nr. 7, 30. Juni 2018 (2018-06-30), Seiten 697-703, XP009513131, ISSN: 1715-5320, DOI: 10.1139/APNM-2017-0547
- FAIR H M ET AL: "Normotensive habitual Caffeine consumers experience no change in blood pressure or pulse wave velocity following the combined consumption of caffeine and dietary nitrate", CIRCULATION, AMERICAN HEART ASSOCIATION, INC, US; AMERICAN HEART ASSOCIATION'S EPIDEMIOLOGY AND PREVENTION/LIFESTYLE AND CARDIOMETABOLIC HEALTH 2019 SCIENTIFIC SESSIONS 20190305 TO 20190308 HOUSTON, TX, Bd. 139, Nr. Supplement 1, 1. März 2019 (2019-03-01), Seiten 1-4, XP009513129, ISSN: 1524-4539, DOI: 10.1161/CIRC.139.SUPPL_1.P298

## Beschreibung

Die vorliegende Erfindung betrifft das medizinische Gebiet der kardiovaskulären Gesundheit.

Insbesondere betrifft die vorliegende Erfindung eine Zusammensetzung, welche eine anregende und konzentrationsfördernde Wirkung besitzt und gleichzeitig zu einer Senkung des arteriellen Blutdrucks führt. Darüber hinaus betrifft die vorliegende Erfindung auch entsprechende Verwendungen der erfindungsgemäßen Zusammensetzungen.

In den Industrieländern stellen kardiovaskuläre Erkrankungen bzw. Erkrankungen des Herz-Kreislaufsystems die häufigste Todesursache dar. In den letzten Jahren lag in Deutschland der Anteil von Herz-Kreislauferkrankungen an den Gesamttodesursachen bei ca. 40 %, bezogen auf das jeweilige Jahr. Die größten Risikofaktoren für die Entwicklung von Herz-Kreislauferkrankungen stellen übermäßiger Tabak- und Alkoholkonsum, Übergewicht, sowie eine ungesunde bzw. unausgewogene Ernährung dar. Auch Bewegungsmangel, erhöhtes LDL-Cholesterin, Diabetes mellitus und Stress stehen in einem engen Zusammenhang mit kardiovaskulären Erkrankungen. Die zuvor genannten Risikofaktoren begünstigen die Entwicklung von arterieller Hypertonie (Bluthochdruck), welche das Herz-/Kreislaufsystem stark belastet und das Risiko für kardiovaskuläre Erkrankungen signifikant steigert.

Normale Blutdruckwerte bei Erwachsenen liegen ungefähr bei 120/80 mmHg ("120 zu 80"), wobei niedrigere Werte grundsätzlich nicht schädlich sind, sofern keine die Lebensqualität einschränkenden Symptome, wie Schwindel, erhöhter Puls, Ohnmacht, Müdigkeit, kalte Hände und Füße sowie Konzentrationsstörungen, auftreten. Von einem niedrigen Blutdruck (Hypotonie) spricht man bei einem Blutdruckwert von weniger als 100/60 mmHg. Ein sehr niedriger Blutdruck kann mit Schwindel, Antriebslosigkeit, Ohnmacht, Müdigkeit und kalten Extremitäten verbunden sein. Von Bluthochdruck bzw. arterieller Hypertonie wird ab einem Blutdruckwert von 140/90 mmHg gesprochen.

Grundsätzlich ist im Hinblick auf die Werte zudem zu beachten, dass der Blutdruck im Alter in der Regel etwas zunimmt, da die Elastizität der Gefäße abnimmt. Leicht über den Normalwerten liegende Blutdruckwerte bei älteren Menschen bzw. Senioren können somit tolerierbar sein, sofern keine weiteren Risikofaktoren für kardiovaskuläre Erkrankungen bestehen. Dennoch sollte auch bei alten Menschen der Blutdruck nicht zu hoch sein, um das Herz-Kreislaufsystem nicht unnötig zu belasten.

Sämtliche der vorgenannten Risikofaktoren begünstigen insbesondere die Entstehung einer Atherosklerose (Arteriosklerose) d. h. einer krankhaften Einlagerung von Cholesterinestern und anderen Fetten in die innere Wandschicht arterieller Blutgefäße, welche wiederum ein Hauptrisikofaktor für Herzinfarkte, Schlaganfälle, Verschlusskrankheiten sowie Aneurysmen ist.

Um einen erhöhten Blutdruck zu senken und so auch das Risiko einer Atherosklerose zu verringern, stehen zunächst nicht-medikamentöse Maßnahmen und Behandlungskonzepte im Vordergrund. Dabei wird versucht, eine Blutdrucksenkung durch eine gesunde Lebensweise auf Basis einer ausgewogenen, vitamin- und ballaststoffreichen Ernährung, insbesondere auch vor dem Hintergrund der Herbeiführung einer Gewichtsabnahme, sowie regelmäßiger Bewegung zu erreichen. In der Regel sind derartige Maßnahmen jedoch nur bei einem leicht erhöhten Blutdruck ausreichend, wohingegen in schwereren Fällen, d. h. ausgeprägter Hypertonie, die erforderliche Blutdrucksenkung auf dieser Basis nicht mehr erzielt werden kann.

Wenn die vorgenannten Änderungen des Lebensstils bzw. der Lebensgewohnheiten nicht ausreichen, um den Blutdruck hinreichend zu senken, ist eine medikamentöse Therapie unausweichlich.

Zur medikamentösen Therapie von Bluthochdruck kommen in der Regel verschiedene blutdrucksenkende Wirkstoffe, zusammenfasst auch als Antihypertensiva bezeichnet, zum Einsatz:
ACE-Hemmer bzw. AT-1-Rezeptor-Antagonisten werden aufgrund ihrer gefäßerweiternden Wirkung eingesetzt, wobei die Wirkung auf der Hemmung des blutdrucksteigernden Hormons Angiotensin II basiert.

Zudem werden Diuretika bzw. entwässernde, harntreibende Mittel eingesetzt, welche die Ausscheidung von Kochsalz und Wasser über die Nieren fördern. In Kombination mit anderen blutdrucksenkenden Medikamenten wird deren Wirkung jeweils verstärkt.

Darüber hinaus kommen häufig sogenannte Betablocker zum Einsatz, welche die Beta-Rezeptoren blockieren, wodurch die Wirkung von Stresshormonen (Noradrenalin, Adrenalin) gehemmt wird. Durch die Blockierung der Wirkung von Stresshormonen sinken Puls und Blutdruck, so dass das Herz insgesamt langsamer schlägt und entlastet wird.

Gleichermaßen kommen auch Alpha-Blocker bzw. Alpha-Rezeptorblocker zum Einsatz, welche gefäßerweiternd und dadurch blutdrucksenkend wirken. Die Wirkung basiert auf einer kompetitiven Hemmung der Alpha1-Adrenorezeptoren.

Schließlich werden auch Calciumantagonisten bzw. Calciumkanalblocker eingesetzt, welche die Calciumkanäle in den Herz- und Gefäßmuskelzellen blockieren, so dass die Gefäßspannung herabgesetzt wird und somit auch der Blutdruck sinkt.

Die vorgenannten Wirkstoffe werden im Rahmen der Behandlung von Hypertonie sowohl als Mono- als auch als Kombinationstherapie verwendet.

Problematisch an den vorgenannten blutdrucksenkenden Medikamenten ist jedoch, dass diese ein hohes Nebenwirkungspotential besitzen. So geht mit ACE-Hemmern beispielsweise oftmals ein unangenehmer Reizhusten einher. Darüber hinaus kann es zur Ausbildung eines Angioödems, d. h. einer Haut- bzw. Schleimhautschwellung, kommen. In seltenen Fällen können auch Nierenfunktionsstörungen auftreten oder verstärkt werden. Calciumantagonisten hingegen können zum sogenannten "Flushing" führen. Dabei rötet sich die Haut und fühlt sich wärmer an, insbesondere im Gesicht. Darüber hinaus kann es zu Kopfschmerzen, Palpitationen sowie Wassereinlagerungen kommen. Die häufig verabreichten Betablocker hingegen können zu einer Verengung der Bronchien führen und zudem die Herzfrequenz verlangsamen (Bradykardie). Zudem kann es auch zu Durchblutungsstörungen in den Extremitäten kommen. Auch sind Müdigkeit, depressive Verstimmungen, Schwindelgefühle und Schlafstörungen möglich. Die Gabe von Diuretika bzw. Entwässerungsmitteln kann zudem zu einer Senkung des Natrium- und/oder Kaliumspiegels im Blut führen, was im schlimmsten Falle in einer lebensbedrohlichen Hyponatriämie oder Hypokaliämie enden kann.

Weiterer Nachteil an einer medikamentösen Therapie von Bluthochdruck ist zudem, dass die Einstellung auf eine geeignete bzw. optimale Wirkstoffmenge bzw. -dosierung langwierig sein kann. Insbesondere bei Überdosierungen kommt es zu einer zu starken Absenkung des Blutdrucks, was mit Schwindel, Konzentrationsstörungen und Antriebslosigkeit verbunden sein kann.

Vor dem Hintergrund der obigen Ausführungen wird ersichtlich, dass im Stand der Technik ein Bedarf an nebenwirkungsarmen und gut verträglichen Mitteln besteht, welche zu einer Senkung des Blutdrucks führen und für die Prävention oder Behandlung von Herz-Kreislauferkrankungen bzw. kardiovaskulären Erkrankungen geeignet sind. Dokument US2018/133247 offenbart die Verwendung einer Zusammensetzung enthaltend rote Bete Extrakt (mit KNO₃ in einer Menge von 171 mg per Tablette) und Caffeoylquinicsäure (mit <2% Koffein, i.e. 2% von 57 mg, i.e. 0.28 mg) in einem Patient und ihres Effekt auf das Halten des arteriellen normalen Blutdrucks (Beispiel 1) und zur Senkung von LDL und des intraokularen Drucks. Auch Vitamin C ist in einer Menge von 60 mg enthalten.

Der vorliegenden Erfindung liegt daher die Aufgabe zugrunde, Zusammensetzungen bzw. Therapiekonzepte bereitzustellen, welche die vorgenannten Nachteile der aus dem Stand der Technik bekannten Konzepte überwinden oder aber zumindest abschwächen.

Insbesondere liegt der vorliegenden Erfindung die Aufgabe zugrunde, Zusammensetzungen bzw. Therapiekonzepte zur Behandlung von Bluthochdruck bzw. zur Vorbeugung von kardiovaskulären Erkrankungen bereitzustellen, welche im Vergleich zum Stand der Technik geringere Nebenwirkungen, vorzugsweise im Wesentlichen keine Nebenwirkungen, aufweisen, und insgesamt in ihren Eigenschaften und Wirkungen verbessert sind.

Zur Lösung der zuvor geschilderten Aufgabe schlägt die vorliegende Erfindung - gemäß dem ersten Aspekt der vorliegenden Erfindung - eine Zusammensetzung nach Anspruch 1 vor; weitere, insbesondere vorteilhafte Ausgestaltungen der erfindungsgemäßen Zusammensetzung sind Gegenstand der diesbezüglichen Unteransprüche.

Weiterhin betrifft die vorliegende Erfindung - gemäß einem zweiten Aspekt der vorliegenden Erfindung - die erfindungsgemäßen Verwendungen der Zusammensetzungen nach der vorliegenden Erfindung.

Es versteht sich von selbst, dass Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht der Fachmann aber auch von selbst.

Weiterhin gilt, dass der Fachmann - anwendungsbezogen oder einzelfallbedingt - von den nachfolgend angeführten Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert. Nur der Gegenstand der Ansprüchen ist Teil der Erfidung. Die andere Ausführungsformen die hier beschrieben werden und die nicht unter der Ansprüchen fallen, sind nicht Teil der Erfindung.

Gegenstand der vorliegenden Erfindung ist somit - gemäß einem **ersten** erfindungsgemäßen Aspekt - eine Zusammensetzung mit konzentrationsfördernder und/oder anregender Wirkung, zur gleichzeitigen Senkung des arteriellen Blutdrucks, wobei die Zusammensetzung
(a) als Komponente (a) mindestens eine Nitratquelle; und
(b) als Komponente (b) mindestens eine Substanz mit stimulierender und/oder anregender Wirkung, nämlich Koffein und Sulforaphan enthält,
   wobei die Komponente (a) und/oder die Nitratquelle Nitrat in einer Menge, bezogen auf die gewichtsbezogene Menge an NO₃ und/oder NO₃⁻ pro Portion und/oder Applikationseinheit der Zusammensetzung, im Bereich 10 bis 5.000 mg enthält,
   wobei die Zusammensetzung die Komponente (b), bezogen auf eine Portion und/oder Applikationseinheit der Zusammensetzung, in einer Menge im Bereich von 10 bis 500 mg enthält; und
   wobei die Zusammensetzung Nitrat und die Komponente (b) in einem gewichtsbezogenen Mengenverhältnis von [gewichtsbezogene Menge N03] : [gewichtsbezogene Menge Komponente (b)] im Bereich von 1 : 2 bis 20 : 1 enthält.

Im Rahmen der vorliegenden Erfindung wurde vollkommen überraschend eine Zusammensetzung gefunden, welche auf Basis der darin enthaltenen Wirkstoffkombination von Nitrat einerseits und einer Komponente mit stimulierender bzw. anregender Wirkung, nämlich Koffein, andererseits eine Steigerung der Konzentration ermöglicht bzw. eine anregende Wirkung entfaltet, gleichzeitig jedoch der arterielle Blutdruck gesenkt werden kann.

Mit anderen Worten ist es mit der erfindungsgemäßen Zusammensetzung gelungen, zwei grundsätzlich verschiedene bzw. sich eigentlich ausschließende Wirkungen in einer einzigen Zusammensetzung zu vereinen. Üblicherweise führen nämlich stimulierende bzw. anregende Wirkstoffe, wie z. B. Koffein, zu einer Erhöhung von Puls und Blutdruck und blutdrucksenkende Mittel wirken gerade nicht konzentrationsfördernd bzw. nicht anregend, sondern vielmehr beruhigend und den Herzschlag verlangsamend.

Die Blutdrucksenkung wird im Rahmen der vorliegenden Erfindung - ohne sich dabei auf diese Theorie beschränken zu wollen - maßgeblich durch das in der erfindungsgemäßen Zusammensetzung enthaltene Nitrat erzielt. Auf Basis einer vorzugsweise oralen Aufnahme von Nitrat bzw. topischen Applikation von Nitrat in der Mundhöhle kann der Blutdruck signifikant gesenkt werden. Die Aufnahme von Nitrat führt zu einer verbesserten Gefäßfunktion, d. h. durch die Aufnahme von Nitrat werden die Blutgefäße, insbesondere die Arterien, geweitet, was wiederum mit einem effizienteren Blutfluss verbunden ist und zu einer Entlastung des Herz-Kreislaufsystems führt.

Die Wirkung von Nitrat als Blutdrucksenker beruht - ebenfalls ohne sich hierbei auf diese Theorie beschränken zu wollen - auf dem NO-Stoffwechsel, welcher zur Reduktion von Nitrat (NO₃⁻) über Nitrit (NO₂⁻) zu Stickstoff-Monoxid (NO) führt. Bei oraler Aufnahme von Nitrat wird dieses bereits im Mund von Bakterien bzw. Mikroorganismen der Mundflora und Mundschleimhaut zu Nitrit reduziert. Nach dem Schlucken des Speichels gelangt das Nitrit in den Blutkreislauf, wo es wiederum enzymatisch durch Nitritreduktasen in Stickstoff-Monoxid umgewandelt wird. Bei Stickstoffmonoxid handelt es sich um ein Signalmolekül, welches im menschlichen Körper eine zentrale Rolle im Hinblick auf die vaskuläre Homöostase spielt. Insbesondere wirkt Stickstoffmonoxid als Vasodilatator und verhindert ein schnelles Wachstum der glatten Gefäßmuskelzellen. Durch einen Anstieg des NO-Spiegels im Blut werden Gefäßverengungen signifikant reduziert. Darüber hinaus wirkt Stickstoffmonoxid auch in der Neurotransmission, Immunabwehr und Hemmung der Aggregation von Thrombozyten mit. Durch die Förderung des NO-Spiegels im Blut kann somit insgesamt eine natürliche Senkung des Blutdrucks erzielt werden.

Die vorliegende Erfindung weist zahlreiche weitere Vorteile und Besonderheiten auf, welche nachfolgend aufgeführt sind und als Indiz für das Vorliegen einer erfinderischen Tätigkeit zu werten sind:

Wie zuvor bereits ausgeführt, war es vollkommen überraschend, dass auf Basis der erfindungsgemäßen Zusammensetzung zwei konträre Wirkungen, nämlich blutdrucksenkend einerseits und anregend bzw. konzentrationsfördernd andererseits, in einer gemeinsamen Zusammensetzung kombiniert werden können. Insbesondere war es in diesem Zusammenhang überraschend, dass die Zugabe einer anregenden, stimulierenden bzw. konzentrationsfördernden Substanz, vorzugsweise Koffein, nicht zu einer Aufhebung der blutdrucksenkenden Wirkung des Nitrats bzw. der Nitratquelle führt. Denn anregende bzw. stimulierende Substanzen, wie Koffein, führen üblicherweise zu einem gegenteiligen Effekt von blutdrucksenkenden Substanzen, nämlich einer Steigerung von Herzschlag und Blutdruck.

Vor dem Hintergrund, dass die erfindungsgemäße Zusammensetzung auf natürlichen, gut verträglichen Inhaltsstoffen basiert, ist die Einnahme der Zusammensetzung zumindest im Wesentlichen nebenwirkungsfrei. Insbesondere treten die bei der medikamentösen Therapie von arterieller Hypertonie möglichen Nebenwirkungen, z. B. Reizhusten durch ACE-Hemmer, Schlappheit, eine verlangsamte Herzfrequenz, Schwindelgefühle, Müdigkeit etc., bei Einnahme der erfindungsgemäßen Zusammensetzung nicht auf. Auf Basis der erfindungsgemäßen Zusammensetzung ist somit eine wirksame und nicht-medikamentöse Behandlung oder Prävention von Bluthochdruck möglich, wobei die bei der medikamentösen Therapie auftretenden Nebenwirkungen vermieden werden und die erfindungsgemäße Zusammensetzung insgesamt eine höhere Verträglichkeit besitzt.

Darüber hinaus besteht bei der erfindungsgemäßen Zusammensetzung keine Gefahr von Fehl- oder Überdosierungen. Denn - wie die nachstehend noch geschilderten Ausführungsbeispiele zeigen - tritt der Effekt der Blutdrucksenkung nur bei Patienten ein, welche tatsächlich einen zu hohen Blutdruck oder einen Blutdruck im Bereich der oberen Normwerte aufweisen. Mit anderen Worten tritt die blutdrucksenkende Wirkung nur bei einem erhöhten basalen Blutdruck auf. Auch führt eine Erhöhung der Dosis/Wirkstoffmenge nicht zu einer weiteren Senkung des Blutdrucks. Dabei wird der Blutdruck jedoch niemals zu stark gesenkt, d. h. es erfolgt keine Senkung in den Bereich eines niedrigen Blutdrucks von weniger als 100/60 mmHg. Bei Einnahme der erfindungsgemäßen Zusammensetzung bei normalem oder niedrigem Blutdruck entfaltet diese also keine weiterführende blutdrucksenkende Wirkung. Auch Personen mit "gesundem Blutdruck" können die erfindungsgemäße Zusammensetzung somit gefahrlos konsumieren und von der konzentrationsfördernden bzw. anregenden Wirkung profitieren, ohne dass der Blutdruck weiter gesenkt würde.

Die erfindungsgemäße Zusammensetzung kann zudem in verschiedensten Darreichungsformen vorliegen, wobei es bevorzugt ist, wenn es sich bei den erfindungsgemäßen Zusammensetzungen um für den Verzehr vorgesehene Zusammensetzungen handelt oder Zusammensetzungen, welche für die topische Applikation in der Mundhöhle hergerichtet sind. Denn, wie zuvor ausgeführt, wird Nitrat bereits in der Mundhöhle durch nitratreduzierende Mikroorganismen und Bakterien der Mundflora zu Nitrit reduziert, welches wiederum mit dem Speichel heruntergeschluckt wird und somit in die Blutbahn zur Generierung von Stickstoffmonoxid gelangt. Besonders gute Ergebnisse werden im Hinblick auf die Wirkungen der erfindungsgemäßen Zusammensetzungen erzielt, wenn die Zusammensetzungen in Form von Getränken vorliegen (vgl. Ausführungsbeispiele). Darüber hinaus ist jedoch auch die Bereitstellung der Zusammensetzungen insbesondere in Form von Kaugummis, Mundspüllösungen bzw. Mundwässern oder Zahncreme gleichermaßen möglich.

Die erfindungsgemäße Zusammensetzung eignet sich somit insbesondere auch für die Verwendung als Nahrungs(ergänzungs)mittel, um nebenwirkungsfrei und gefahrlos die kardiovaskuläre Gesundheit zu unterstützen und den Blutdruck zu senken bzw. einem zu hohen Blutdruck vorzubeugen.

Im Hinblick auf die Wirkungen der erfindungsgemäßen Zusammensetzungen wird bereits an dieser Stelle auf die nachfolgend noch im Detail geschilderten Ausführungsbeispiele verwiesen, welche die Wirkungen der erfindungsgemäßen Zusammensetzungen, insbesondere im Hinblick auf die Senkung des Blutdrucks, belegen und als Indiz für das Vorliegen einer erfinderischen Tätigkeit zu werten sind.

Nachfolgend werden zum besseren Verständnis der erfindungsgemäßen Zusammensetzung bzw. des erfindungsgemäßen Konzepts Definitionen der im Rahmen der vorliegenden Erfindung verwendeten Begrifflichkeiten gegeben und darüber hinaus besonders bevorzugte Ausführungsformen der vorliegenden Erfindung beschrieben.

Der Begriff "Zusammensetzung", wie er im Rahmen der vorliegenden Erfindung verwendet wird, ist sehr umfassend zu verstehen und bezeichnet auf der einen Seite pharmazeutische bzw. medizinische Präparate und auf der anderen Seite auch Lebensmittelzusammensetzungen, wie z. B. Getränke oder sonstige für den oralen Verzehr vorgesehene Nahrungs(ergänzungs)mittel, darüber hinaus jedoch auch Kosmetika bzw. Hygieneartikel oder Drogerieprodukte, wie z. B. Zahn-/ Mundpflegeprodukte, wie Kaugummis oder Mundspüllösungen.

Der Begriff "Nitratquelle" bezeichnet im Rahmen der vorliegenden Erfindung Substanzen, welche für den Verzehr geeignet bzw. pharmazeutisch verträglich sind und Nitrate, insbesondere anorganisches Nitratsalz oder organische Nitrate, enthalten, wie z. B. Pflanzen, Gemüse etc. mit hohem Nitratgehalt. Gleichermaßen kann eine Nitratquelle im Sinne der vorliegenden Erfindung auch durch Nitrate, insbesondere anorganisches Nitratsalz oder organische Nitrate, als solche gebildet sein. Im Rahmen der vorliegenden Erfindung ist es besondere bevorzugt, wenn die Nitratquelle pflanzlichen Ursprungs ist, d. h. als Nitratquelle Bestandteile von Pflanzen eingesetzt, welche Nitrat anreichern bzw. speichern. Zu den Pflanzen, welche große Mengen an Nitrat enthalten, gehören unter anderem Rüben (Bete), Mangold, Spinat, diverse Salate, Kresse und Kohlsorten. Wie nachfolgend noch ausgeführt, können pflanzliche Nitratquellen in Form von flüssigen Bestandteilen der Pflanzen, insbesondere den aus Pflanzen oder Pflanzenbestandteilen gewonnenen Säften oder Extrakten, eingesetzt werden. Darüber hinaus ist es auch möglich, feste Pflanzenbestandteile, beispielsweise gemahlene und pulverisierte Bestandteile, wie z. B. Wurzeln, Blätter oder Speicherorgane, als Nitratquelle einzusetzen.

Was den Begriff "Nitrat" anbelangt, so werden im Rahmen der vorliegenden Erfindung hierunter die Salze und Ester der Salpetersäure (HNO₃) verstanden, wobei Nitratsalze die allgemeine Zusammensetzung M^{l}NO₃ aufweisen (M^{l}: einwertiges Kation). Üblicherweise handelt es sich bei dem einwertigen Kation um ein Alkalisalz, insbesondere Kalium oder Natrium. Gleichermaßen können auch Nitratsalze auf Basis von Nitrat-Anionen und Erdalkalimetallen, insbesondere Magnesium, Kalzium oder Barium, gebildet sein. Bei Estern der Salpetersäure handelt es sich um organische Nitratverbindungen, welche die allgemeine Struktur R-O-NO₂ (R: organischer Rest) aufweisen. Für weitergehende Einzelheiten zu dem Begriff "Nitrat" wird auf RÖMPP Chemie Lexikon, 10. Auflage (1998), Georg Thieme Verlag, Stichwort: "Nitrate", Seite 2911, verwiesen, wobei der darin beschriebene Offenbarungsgehalt hiermit durch Bezugnahme eingeschlossen wird.

Unter dem Begriff einer "stimulierenden bzw. anregenden Substanz" werden insbesondere psychotrope Substanzen verstanden, welche die Aktivität der Nerven erhöhen, beschleunigen oder verbessern, so dass Konzentration, Wachheit und Energielevel gesteigert werden.

Erfindungsgemäß ist es besonders bevorzugt, wenn als stimulierende bzw. anregende Substanz Koffein eingesetzt wird. Bei Koffein (synonym auch als Coffein, Tein, Teein oder Thein bezeichnet) handelt es sich um ein Alkaloid aus der Stoffgruppe der Xanthine. Die chemische Bezeichnung von Koffein lautet 1,3,7-Trimethyl-2,6-Purindion bzw. 1,3,7-Trimethylxanthin. Koffein ist eine psychoaktive Substanz mit stimulierender Wirkung und wird in erster Linie aus den Bohnen von Kaffeepflanzen (*Coffea*) oder Blättern des Teestrauchs (*Camellia sinensis*) gewonnen. Das Wirkspektrum von Koffein ist sehr breit, wobei die wesentlichen Wirkungen von Koffein in einer Anregung des Zentralnervensystems, einer Erhöhung der Kontraktionskraft des Herzens, in einer Pulssteigerung, Bronchialerweiterung, leichter Erhöhung des Blutdrucks und Anregung der Peristaltik des Darmes bestehen. Bei Einsatz relativ geringer Dosen von Koffein steht jedoch maßgeblich die stimulierende Wirkung auf Basis der Anregung des Zentralnervensystems im Vordergrund. Durch die Anregung des Zentralnervensystems lassen sich mit dem Einsatz von Koffein Aufmerksamkeit und Konzentrationsvermögen erhöhen.

Wie zuvor bereits ausgeführt, wird mit der erfindungsgemäßen Wirkstoffkombination eine Zusammensetzung bereitgestellt, welche in vollkommen überraschender Weise zwei gegensätzliche Wirkungen bzw. Effekte in einer einzigen Zusammensetzung vereint, nämlich eine konzentrationsfördernde und anregende Wirkung einerseits und eine Senkung des arteriellen Blutdrucks andererseits.

Die erfindungsgemäße Zusammensetzung kann in vielfältiger Weise ausgestaltet sein, wobei bevorzugte Ausführungsformen im Nachfolgenden geschildert sind.

Was die eingesetzte Menge an Nitrat in den erfindungsgemäßen Zusammensetzungen anbelangt, kann diese in weiten Bereichen variieren, im Bereich von 10 bis 1000 mg. Im Hinblick auf eine gute Wirksamkeit bei der Senkung des Blutdrucks hat es sich als vorteilhaft erwiesen, wenn die Komponente (a) und/oder die Nitratquelle Nitrat in einer Menge, bezogen auf die gewichtszogene Menge an NO₃ und/oder NO₃⁻ pro Portion und/oder Applikationseinheit der Zusammensetzung, im Bereich von 10 bis 5.000 mg, vorzugsweise 100 bis 2.500 mg, bevorzugt 200 bis 1.000 mg, besonders bevorzugt 300 bis 700 mg, enthält.

Mit einer Nitratsupplementation bzw. Applikation von Nitrat in der Mundhöhle in den vorgenannten Mengenbereichen kann eine Senkung des systolischen Blutdrucks von bis zu 15 mmHg erzielt werden. Wie zuvor bereits ausgeführt, ist die Wirkung des Nitrats vom basalen Blutdruck abhängig, d. h. je höher der Ausgangsblutdruck ist, desto besser ist die blutdrucksenkende Wirkung der erfindungsgemäßen Zusammensetzungen. Ist der Blutdruck hingegen niedrig bis normal, wird durch die erfindungsgemäßen Zusammensetzungen der Blutdruck nicht weiter gesenkt.

Im Rahmen der vorliegenden Erfindung kann es vorgesehen sein, dass die Komponente (a) und/oder die mindestens eine Nitratquelle aus pflanzlichen Nitratquellen und/oder Salzen und/oder Estern, insbesondere Salzen, der Salpetersäure und/oder deren Mischungen, vorzugsweise pflanzlichen Nitratquellen, ausgewählt ist.

Gemäß einer besonders bevorzugten Ausführungsform ist es im Hinblick auf die Komponente (a) also vorgesehen, dass diese auf einer Nitratquelle pflanzlichen Ursprungs basiert, d. h. die Zusammensetzung als Komponente (a) mindestens eine pflanzliche Nitratquelle enthält. Gemäß dieser speziellen Ausführungsform der vorliegenden Erfindung kann die Nitratquelle insbesondere aus Pflanzen der Gruppe von *Spinacia oleracea* (Gemüsespinat), *Raphanus* (Rettich), *Beta vulgaris* (Gemeine Rübe), *Brassica rapa* (Speiserübe), *Diplotaxis tenuifolia* (Rucola), *Lepidium* (Kresse), *Valerianella* (Feldsalat), vorzugsweise *Beta vulgaris* (Gemeine Rübe), ausgewählt sein. Die vorgenannten Pflanzen, insbesondere ihre Blätter und Speicherorgane, sind reich an Nitrat und können einen Nitratgehalt von mehr als 1.000 mg/kg aufweisen.

Besonders gute Ergebnisse werden erzielt, wenn die pflanzliche Nitratquelle auf Bestandteilen von *Beta vulgaris* basiert, welche diverse Subspezies und Kulturformen umfasst. *Beta vulgaris* bzw. die Gemeine Rübe ist eine Pflanzenart aus der Familie der Fuchsschwanzgewächse mit zahlreichen Subspezies, zu welchen unter anderem die Zuckerrübe, verschiedene Kulturformen der Bete, Mangold und die Futterrübe gehören. Die Gemeine Rübe stammt von der wilden Rübe ab, welche ein weitreichendes Verbreitungsgebiet von Westeuropa bis nach Westasien besitzt. Im Hinblick auf die vorliegende Erfindung ist der Einsatz von *Beta vulgaris* auf Basis der Kulturformen "Rote Bete", "Weiße Bete" bzw. "Gelbe Bete" besonders bevorzugt.

Der Gehalt an Nitrat, insbesondere in den Speicherorganen, liegt in der Regel im Bereich von 1.000 bis 4.000 mg/kg. Die vorgenannten Kulturformen der Bete weisen zudem einen hohen Vitamin B-, Kalium-, Eisen- und Folsäuregehalt auf.

Gemäß einer besonders bevorzugten Ausführungsform der erfindungsgemäßen Zusammensetzung kann es somit vorgesehen sein, dass die Zusammensetzung als Komponente (a) mindestens eine pflanzliche Nitratquelle enthält, wobei die pflanzliche Nitratquelle aus der gelben, roten und/oder weißen Kulturform, bevorzugt aus der gelben und/oder weißen Kulturform, der Gemeinen Rübe (*Beta vulgaris*) gewonnen ist. Im Hinblick auf die blutdrucksenkende Wirkung sind die vorgenannten Sorten von Bete gleichermaßen geeignet. Der Einsatz speziell von hellen Beten, d. h. Gelber oder Weißer Bete, hat sich jedoch dahingehend als vorteilhaft erwiesen, als diese im Wesentlichen frei von dem tiefroten Naturfarbstoff Betanin sind. Nachteilig an dem Farbstoff ist nämlich, dass dieser bei Verzehr in den Urin gelangen kann und zu einer roten Einfärbung des Urins führt. Problematisch an durch Betanin rot eingefärbtem Urin ist zum einen, dass eine Labordiagnostik des Urins eines Patienten erschwert wird, da Betanin die chemisch-biologische Analyse stört. Zum anderen führt rot eingefärbter Urin teilweise zu Verunsicherungen bei Patienten bzw. Konsumenten der Zusammensetzungen, da diesen nicht immer bewusst ist, dass die Einfärbung harmlos und lediglich auf den in Bete enthaltenen Farbstoff zurückzuführen ist und es sich nicht um Blut oder sonstige Krankheitssymptome handelt. Insofern ist es im Rahmen der vorliegenden Erfindung besonders bevorzugt, wenn die pflanzliche Nitratquelle auf Gelber und/oder Weißer Bete basiert.

Was die erfindungsgemäße Ausführungsform, wonach die Komponente (a) auf mindestens einer pflanzlichen Nitratquelle basiert, anbelangt, so ist es besonders bevorzugt, wenn die Zusammensetzung die mindestens eine pflanzliche Nitratquelle in Form von flüssigen Pflanzenbestandteilen, insbesondere Pflanzenextrakt und/oder Pflanzensaft, und/oder in Form von festen Pflanzenbestandteilen, insbesondere Blättern, Wurzeln und/oder Speicherorganen in vorzugsweise zermahlener Form, enthält. Besonders gute Ergebnisse werden insbesondere im Hinblick auf die Bereitstellung von Zusammensetzungen in flüssiger Form, wie z. B. Getränken, erzielt, wenn die pflanzliche Nitratquelle in Form von Pflanzenextrakt bzw. Pflanzensaft eingesetzt wird. Gemäß einer besonders bevorzugten Ausführungsform der vorliegenden Erfindung kann es dabei vorgesehen sein, dass die flüssige Nitratquelle, z. B. Bete-Saft, die Basis für die Zusammensetzung darstellt (vgl. auch erfindungsgemäße Ausführungsbeispiele), welcher dann die übrigen Inhaltsstoffe zugemischt werden.

Gemäß einer weiteren Ausführungsform der vorliegenden Erfindung kann es zudem vorgesehen sein, dass die Zusammensetzung als Komponente (a) Nitrat in Form von Salzen und/oder Estern der Salpetersäure, insbesondere Salzen der Salpetersäure, enthält. In diesem Zusammenhang ist es besonders bevorzugt, wenn die Komponente (a) ausgewählt ist aus der Gruppe der Alkali- und/oder Erdalkalisalze der Salpetersäure, insbesondere Kaliumnitrat, Natriumnitrat, Bariumnitrat, Calciumnitrat, Magnesiumnitrat und/oder deren Mischungen, bevorzugt Kaliumnitrat und/oder Natriumnitrat.

Gemäß einer weiteren Ausführungsform kann es zudem vorgesehen sein, dass die erfindungsgemäße Zusammensetzung als Komponente (a) eine Kombination von einer Nitratquelle pflanzlichen Ursprungs, wie z. B. Bete-Saft, und anorganischem Nitrat, z. B. auf Basis von Kalium- oder Natriumnitrat, enthält.

Was die Komponente (b) auf Basis einer Substanz mit stimulierender bzw. anregender Wirkung anbelangt, ist als Komponente (b) Koffein enthalten.

Die eingesetzte Menge an Koffein ist dabei variabel im Bereich von 10 bis 500 mg. Besonders gute Ergebnisse werden erzielt, wenn die Zusammensetzung die Komponente (b), bezogen auf eine Portion und/oder Applikationseinheit der Zusammensetzung, in einer Menge im Bereich von 10 bis 500 mg, bevorzugt 50 bis 250 mg, besonders bevorzugt 80 bis 150 mg, enthält. Auf Basis der vorgenannten Mengenangaben wird eine das Zentralnervensystem stimulierende Wirkung erzielt, insbesondere die Konzentrationsfähigkeit erhöht, ohne jedoch die Wirkung des Nitrats als Blutdrucksenker aufzuheben.

Um die Wirksamkeit bzw. das Wirkspektrum der erfindungsgemäßen Zusammensetzung weiterhin zu verbessern, kann es zudem vorgesehen sein, dass die erfindungsgemäße Zusammensetzung als Komponente (c) mindestens einen Mineralstoff enthält.

Der Begriff der "Mineralstoffe" wird im Rahmen der vorliegenden Erfindung als Sammelbezeichnung für die mineralischen Bestandteile von pflanzlichen und tierischen Organismen, d. h. insbesondere des Menschen, verwendet. Beim menschlichen Körper beträgt die Menge an Mineralstoffen ca. 3,5 kg. Dabei wird zwischen sogenannten Mikroelementen mit Spurenelement-Charakter, welche hauptsächlich eine katalytische Funktion ausüben, und den mengenmäßig weit überwiegenden Makroelementen, die als "Baustoffe", beispielsweise für das Skelett, unentbehrlich sind, unterschieden. Zu den Mikroelementen gehören insbesondere Fluor, Brom, lod, Eisen, Kupfer, Mangan, Kobalt, Zink, Molybdän, Vanadium und Selen. Zu den Makroelementen hingegen zählen Calcium, Natrium, Kalium, Phosphor, Schwefel, Magnesium und Chlor. Für weitergehende Einzelheiten zu dem Begriff der "Mineralstoffe" wird zudem verwiesen auf RÖMPP Chemie Lexikon, 10. Auflage (1998), Georg Thieme Verlag, Stuttgart New York, Stichwort: "Mineralstoffe", Seiten 2700 bis 2701. Gemäß einer bevorzugten Ausführungsform der vorliegenden Erfindung kann es somit im Hinblick auf den Einsatz eines Mineralstoffs vorgesehen sein, dass die Komponente (c) ausgewählt ist aus der Gruppe von Calcium, Chlor, Kalium, Magnesium, Natrium, Phosphor, Schwefel, Eisen, lod, Kupfer, Mangan, Molybdän, Selen, Zink und/oder deren Mischungen, vorzugsweise Magnesium.

Bei Magnesium handelt es sich um ein Erdalkalimetall, welches - wie zuvor ausgeführt - für den Menschen ein Makroelement darstellt. Der Körper eines erwachsenen Menschen enthält ca. 20 g Magnesium. Magnesium ist im menschlichen Körper an zahlreichen enzymatischen Reaktionen beteiligt; entweder als Enzymbestandteil oder als Coenzym. Zudem spielen freie Magnesium-Ionen eine Rolle für die Reizweiterleitung in bzw. an den Nervenzellen, da sie das Potential an den Zellmembranen beeinflussen, und fungieren zudem als sogenannte "*second messenger*" im Immunsystem. Magnesiummangel kann mit verschiedenen Symptomen, wie Ruhelosigkeit, Nervosität, Reizbarkeit, Konzentrationsmangel, Müdigkeit, einem allgemeinen Schwächegefühl, Kopfschmerzen, Herzrhythmusstörungen und Muskelkrämpfen, einhergehen. Durch den Einsatz von Magnesium in den erfindungsgemäßen Zusammensetzungen kann somit einem Magnesiummangel vorgebeugt werden bzw. dieser auch behandelt werden.

Was die eingesetzte Menge an Komponente (c) bzw. des mindestens einen Mineralstoffs anbelangt, so kann diese ebenfalls in weiten Bereichen variieren. Gemäß einer bevorzugten Ausführungsform der vorliegenden Erfindung kann es vorgesehen sein, dass die Zusammensetzung die Komponente (c), bezogen auf eine Portion und/oder Applikationseinheit der Zusammensetzung, in einer Menge im Bereich von 1 bis 1.000 mg, insbesondere 5 bis 500 mg, vorzugsweise 10 bis 250 mg, bevorzugt 50 bis 200 mg, enthält.

Gemäß einer weiteren bevorzugten Ausführungsform der vorliegenden Erfindung kann es zudem vorgesehen sein, dass die Zusammensetzung als Komponente (d) mindestens ein Vitamin und/oder Provitamin enthält.

Unter Vitaminen werden in der Regel organische Verbindungen verstanden, welche dem Organismus nicht direkt als Energieträger dienen, sondern die er vielmehr im Hinblick auf andere Funktionen benötigt. Nahezu alle Vitamine kann der menschliche Organismus jedoch selbst nicht synthetisieren. Stattdessen müssen Vitamine bzw. Vorstufen von Vitaminen (sogenannte Provitamine) mit der Nahrung aufgenommen werden und gehören somit zu den essentiellen Stoffen. Im Falle von Provitaminen bzw. Vorstufen werden diese erst nach ihrer Aufnahme in die physiologisch aktive Wirkform umgewandelt. Grundsätzlich werden Vitamine in fettlösliche (lipophile) und wasserlösliche (hydrophile) Vitamine unterschieden. Was die Funktion von Vitaminen anbelangt, so sind diese an zahlreichen Reaktionen des Stoffwechsels beteiligt. Insbesondere regulieren Vitamine die Verwertung von Kohlenhydraten, Proteinen und Mineralstoffen, so dass Vitamine zumindest indirekt auch an der Energiegewinnung beteiligt sind. Weiterhin wird durch Vitamine das Immunsystem unterstützt. Darüber hinaus wirken Vitamine beim Aufbau von Zellen, Blutkörperchen, Knochen und Zähnen mit. Insgesamt handelt es sich bei Vitaminen um eine chemisch gesehen äußerst heterogene Gruppe deren Mitglieder sich auch hinsichtlich ihrer Funktion und Wirkung deutlich voneinander unterscheiden. Insgesamt sind 13 Vitamine bekannt, nämlich Vitamin A, die B-Vitamine B1, B2, B3, B5, B6, B9 und B12 (nachfolgend zusammengefasst als Vitamin B bezeichnet), Vitamin C, Vitamin D sowie die Vitamine E und K.

Im Rahmen der vorliegenden Erfindung kann es somit insbesondere vorgesehen sein, dass die Komponente (d) ausgewählt ist aus der Gruppe von Vitamin A, Vitamin B, Vitamin C, Vitamin D, Vitamin E, Vitamin K und/oder deren Mischungen, insbesondere Vitamin C.

Bei Vitamin C (synonym auch als Ascorbinsäure bezeichnet) handelt es sich um eine organische Säure mit der chemischen Bezeichnung 5-1,2-Dihydroxyethyl-3,4-Dihydroxy-5-Hydrophoran-2-ON bzw. 3-Oxo-L-Gulonsäure-γ-lacton. Vitamin C wirkt aufgrund seiner antioxidativen Wirkung physiologisch als Radikalfänger. Vitamin C wirkt somit im Körper als Reduktionsmittel und kann auf diese Weise reaktive Sauerstoffspezies (ROS) bzw. freie Radikale inaktivieren, was zu einem verringerten Risiko von oxidativem Stress führt. Übermäßiger oxidativer Stress durch freie Radikale beschleunigt zum einen Alterungsprozesse und begünstigt zum anderen die Entstehung verschiedener Krankheiten. Ein Mangel an Vitamin C ist zudem mit der Vitaminmangelkrankheit Skorbut assoziiert. Durch den Einsatz von Vitamin C in den erfindungsgemäßen Zusammensetzungen wird die Wirkweise der erfindungsgemäßen Zusammensetzung noch weiterführend erweitert und verbessert, da Vitamin C - wie zuvor ausgeführt - als Radikalfänger wirkt und zudem der Vitaminmangelkrankheit Skorbut vorbeugt.

Was darüber hinaus die eingesetzte Menge an Vitaminen bzw. Komponente (d) in den erfindungsgemäßen Zusammensetzungen anbelangt, so kann auch diese in weiten Bereichen variieren. Besonders gute Ergebnisse werden jedoch erzielt, wenn die Zusammensetzung die Komponente (d), bezogen auf eine Portion und/oder Applikationseinheit der Zusammensetzung, in einer Menge im Bereich von 1 bis 5.000 mg, insbesondere 5 bis 2.500 mg, vorzugsweise 10 bis 1.500 mg, bevorzugt 50 bis 1.000 mg, besonders bevorzugt 100 bis 500 mg, noch weiter bevorzugt 200 bis 400 mg, enthält.

Um das Wirkspektrum der erfindungsgemäßen Zusammensetzung weiterführend zu verbessern bzw. eine noch breitere Wirkweise zu erzielen, kann es zudem vorgesehen sein, dass die erfindungsgemäße Zusammensetzung als Komponente (e) mindestens eine Substanz mit antioxidativer Wirkung, vorzugsweise indirekter antioxidativer Wirkung, enthält. Wie zuvor ausgeführt, handelt es sich bei Antioxidantien bzw. Antioxidationsmitteln um chemische Verbindungen, welche eine Oxidation anderer Substanzen verlangsamen oder verhindern. Auf dieser Basis wirken Antioxidantien als Radikalfänger, welche reaktive Sauerstoffspezies, wie z. B. freie Radikale, inaktivieren und auf dieser Basis oxidativen Stress vermeiden bzw. reduzieren. Antioxidantien können jedoch auf unterschiedliche Art und Weise wirken, nämlich durch eine direkte Neutralisierung reaktiver Sauerstoffspezies oder freier Radikale oder indirekt, indem Substanzen zur direkten Neutralisierung der freien Radikale bzw. reaktiven Sauerstoffspezies aktiviert werden. Während Antioxidantien auf Basis von Vitaminen, wie oben bereits ausgeführt, direkt auf freie Radikale wirken, führen indirekte Antioxidantien zu einer Aktivierung der körpereigenen Abwehrmechanismen im Hinblick auf die Vermeidung von oxidativem Stress.

In diesem Zusammenhang hat es sich als notwendig gemäss der Erfindung erwiesen, wenn die Komponente (e) bzw. die Substanz mit antioxidativer Wirkung Sulforaphan wie beansprucht, ist. Bei Sulforaphan, chemisch 1-Isothiocyanato-4-methylsulfinyl-butan, handelt es sich um ein Isothiocyanat, welches bei der enzymatischen Hydrolyse des Senfölglykosids Glucoraphanin entsteht. Zudem kommt Sulforaphan als sekundärer Pflanzenstoff auf Basis von Senfölglykosid-Glucoraphanin insbesondere in Kreuzblütengewächsen (*Brassicaceae*) vor. Sulforaphan wirkt physiologisch als starkes indirektes Antioxidans. Im Körper des Menschen werden durch Sulforaphan sogenannte Phase-II-Enzyme aktiviert, welche wiederum antioxidative Abwehrmechanismen anschieben. Durch den zusätzlichen Einsatz von Sulforaphan in den erfindungsgemäßen Zusammensetzungen kann somit eine eigenständige antioxidative Wirkung erzielt werden oder aber, sofern auch der Einsatz von Vitaminen, insbesondere Vitamin C, vorgesehen ist, die direkte antioxidative Wirkung der Vitamine durch ein zusätzliches indirektes Antioxidans synergistisch ergänzt bzw. verstärkt werden.

Im Hinblick auf die Menge an Komponente (e) bzw. Sulforaphan hat es sich zudem als vorteilhaft erwiesen, wenn die Zusammensetzung die Komponente (e) und/oder Sulforaphan, bezogen auf eine Portion und/oder Applikationseinheit der Zusammensetzung, in einer Menge im Bereich von 0,01 bis 500 mg, insbesondere 0,1 bis 250 mg, vorzugsweise 1 bis 100 mg, bevorzugt 5 bis 50 mg, besonders bevorzugt 10 bis 30 mg, enthält.

Wie zuvor ausgeführt, kann die erfindungsgemäße Zusammensetzung in unterschiedlichsten Darreichungsformen vorliegen. Erfindungsgemäß ist es besonders bevorzugt, wenn die Zusammensetzung für den Verzehr aufbereitet ist oder in Form einer in der Mundhöhle zu applizierenden Zusammensetzung, wie z. B. Mundwasser, Zahnpasta oder Kaugummi, vorliegt. Denn - wie zuvor ausgeführt - beginnt die Umwandlung von Nitrat zu Nitrit bereits in der Mundhöhle durch nitratreduzierende Mikroorganismen.

Je nach Darreichungsform können die Zusammensetzungen somit unterschiedlich ausgestaltet sein und dementsprechend unterschiedliche Hilfsstoffe enthalten, wie nachfolgend ausgeführt:
Im Hinblick auf eine kaubare, jedoch nicht zum Herunterschlucken vorgesehene Zusammensetzung bzw. eine Zusammensetzung in Form von Kaugummi ist es vorteilhaft, wenn die Zusammensetzung als Hilfsstoff Kaugummigrundmasse enthält. Grundsätzlich weiß der Fachmann bei der Bereitstellung einer kaugummiförmigen Zusammensetzung, welche Hilfsstoffe bzw. Grundstoffe dafür einzusetzen sind. Gemäß einer diesbezüglich bevorzugten Ausführungsform kann es vorgesehen sein, dass die Kaugummigrundmasse natürliche Polymere, insbesondere Kautschuk, vorzugsweise in Form von Latex, Crepe oder Sheets, Polyole sowie deren Mischungen, enthält. Gleichermaßen kann es vorgesehen sein, dass die Kaugummigrundmasse synthetische Polymere, insbesondere synthetische Thermoplaste, vorzugsweise Polyvinylether, insbesondere Poly(ethylvinylether) und/oder Poly(isobutylvinylether), Polyvinylacetate, Vinylacetate, Polyisobutene und/oder deren Mischungen, enthält.

Bei der Bereitstellung der erfindungsgemäßen Zusammensetzung in Form von Kaugummis bzw. kaubaren Zusammensetzungen wird das Nitrat bzw. die Nitratquelle in die Kaugummigrundmasse eingelagert bzw. die Kaugummigrundmasse mit Nitrat bzw. der Nitratquelle beschichtet, welches dann beim Kauen in der Mundhöhle freigesetzt wird. Wie zuvor bereits ausgeführt, wird das freigesetzte Nitrat durch die in der Mundflora vorhandenen Mikroorganismen bzw. Bakterien zu Nitrit reduziert und mit dem Speichel heruntergeschluckt, um auf dieser Basis in die Blutbahn zu gelangen, wo schließlich durch Nitritreduktasen die Reduktion von Nitrit zu Stickstoffmonoxid erfolgt.

Gleichermaßen - gemäß einer weiteren bevorzugten Ausführungsform - kann es auch vorgesehen sein, dass die erfindungsgemäße Zusammensetzung in Form von festen Zusammensetzungen, wie Lutschtabletten, Bonbons, Hartkaramellen, Lollies oder dergleichen, vorliegt. In diesem Zusammenhang ist es vorteilhaft, wenn die Zusammensetzung als Hilfsstoff mindestens ein festes Träger- und/oder Matrixmaterial enthält. Die Bereitstellung von lutschtablettenförmigen Zusammensetzungen bzw. Bonbons stellt den Fachmann ebenfalls vor keine technischen Hürden, d. h. er weiß die geeigneten Träger bzw. Matrixmaterialien auszuwählen.

Besonders bevorzugt ist es in diesem Zusammenhang jedoch, wenn für feste Darreichungsformen, insbesondere auf Basis von Bonbons, Hartkaramellen, Lutschtabletten und dergleichen, das Träger- und/oder Matrixmaterial aus Zuckern und/oder Zuckeraustauschstoffen, vorzugsweise aus der Gruppe von Saccharose, Glucose, Dextrose, Fructose, Mannit, Xylit, Sorbit, Isomalt, Maltitsirup, Lactit, Leucrose, Fructooligosacchariden, Glucanen, Polyglucose und/oder deren Mischungen, ausgewählt ist.

Auch in diesem Fall sind die Wirksubstanzen der erfindungsgemäßen Zusammensetzung in das Träger- bzw. Matrixmaterial eingelagert und werden beim Lutschen oder Kauen der Zusammensetzung in der Mundhöhle freigesetzt, zu Nitrit reduziert und mit dem Speichel heruntergeschluckt, so dass eine weiterführende Reduktion zu dem eigentlich den Blutdruck senkenden Stickstoffmonoxid erfolgt.

Schließlich kann es auch vorgesehen sein, dass die erfindungsgemäße Zusammensetzung in flüssiger Darreichungsform vorliegt, wobei der Begriff der flüssigen Darreichungsformen breit zu verstehen ist. Zum einen können hierunter Getränke, wie z. B. "Energydrinks" oder Saftzubereitungen, verstanden werden. Zum anderen umfasst der Begriff der flüssigen Darreichungsformen auch Kosmetika bzw. Hygiene- oder Drogerieartikel, wie z. B. Mundwässer, Mundspüllösungen und dergleichen.

In diesem Zusammenhang ist es bevorzugt, wenn die Zusammensetzung mindestens ein flüssiges Trägermaterial enthält. Gemäß dieser Ausführungsform der vorliegenden Erfindung liegen die Wirk- bzw. Inhaltsstoffe der erfindungsgemäßen Zusammensetzung gelöst oder solubilisiert in dem Trägermaterial vor. Grundsätzlich kommen hier alle pharmazeutisch bzw. ernährungswissenschaftlich verträglichen Trägermaterialien in Betracht.

Wie zuvor im Zusammenhang mit der Nitratquelle ausgeführt, ist es möglich, dass das Trägermaterial für flüssige Zusammensetzungen bereits durch eine flüssige Nitratquelle, wie z. B. Bete-Saft, gebildet ist, welchem dann noch die weiteren Inhaltsstoffe zugesetzt werden. Dies hat sich insbesondere bei der Bereitstellung von trinkbaren Zusammensetzungen als vorteilhaft erwiesen.

Darüber hinaus kann es im Zusammenhang mit weiteren flüssigen Zusammensetzungen, z. B. in Form von Mundwasser, zudem vorgesehen sein, dass das Trägermaterial polare und/oder apolare Lösungsmitteln enthält, vorzugsweise Wasser, Ethanol, Glycerin, hydriertes Rizinusöl (*hydrogenated castor oil*), Polyethylenglykol, Polypropylenglycol und/oder deren Mischungen.

Schließlich kann es im Hinblick auf die vorliegende Erfindung auch vorteilhaft sein, wenn die Zusammensetzung mindestens einen weiteren Inhaltsstoff enthält, insbesondere wobei der mindestens eine weitere Inhaltsstoff ausgewählt ist aus der Gruppe von Zusatz- und/oder Hilfsstoffen, vorzugsweise Binde-, Netz-, Stabilisierungs-, Färbe-, Puffer-, Riech-, Geschmacks-, Süßungs-, Aroma-, Verarbeitungshilfs- und Konservierungsstoffen und -mitteln und/oder deren Mischungen.

Der Einsatz weiterer Inhaltsstoffe kann insbesondere im Hinblick auf den Geschmack der erfindungsgemäßen Zusammensetzung vorgesehen sein, um bestimmte Aromen bzw. Geschmacksrichtungen zu erzeugen oder unerwünschte Geschmäcker, wie sie z. B. mit pflanzlichen Nitratquellen einhergehen können, zu maskieren. Darüber hinaus können weitere Inhaltsstoffe enthalten sein, um Konsistenz, Haltbarkeit, Stabilität etc. der erfindungsgemäßen Zusammensetzung weiterführend zu verbessern.

Wie zuvor bereits ausgeführt, ist es erfindungsgemäß bevorzugt, wenn die Zusammensetzung für den Verzehr und/oder für die topische Applikation, insbesondere in der Mundhöhle, hergerichtet ist.

Auf Basis solcher Zusammensetzungen wird eine Reduktion von Nitrat zu Nitrit bereits in der Mundhöhle gewährleistet, wobei das dabei entstehende Nitrit mit dem Speichel heruntergeschluckt und in der Blutbahn weiterführend zu Stickstoffmonoxid reduziert wird. Bei für den Verzehr vorgesehenen Zusammensetzungen wird zudem auch heruntergeschlucktes Nitrat weiterführend im Körper zu Nitrit umgewandelt, insbesondere nitratreduzierende Bakterien im Magen-/Darmtrakt, und gelangt gleichermaßen zur weiteren Reduktion zu Stickstoffmonoxid in die Blutbahn.

Was die Konsistenz der erfindungsgemäßen Zusammensetzung anbelangt, so ist die Zusammensetzung vorzugsweise in fester, flüssiger, viskoser (gelförmiger), halbfester, aerosolförmiger oder pastöser Form, insbesondere flüssiger oder fester Form, hergerichtet.

Der Fachmann weiß grundsätzlich, wie Zusammensetzungen in den unterschiedlichen Formen bereitgestellt werden können, welche Zusatz- bzw. Hilfsstoffe dazu einzusetzen und welche technischen Maßnahmen zu treffen sind.

Im Hinblick auf konkrete Darreichungsformen kann es gemäß einer bevorzugten Ausführungsform der erfindungsgemäßen Zusammensetzung zudem vorgesehen sein, dass die Zusammensetzung als Getränk, Riegel, Gebäckstück, Hartkaramelle, Kaugummi, Zahnpasta und/oder Mundwasser (Mundspüllösung), insbesondere als Getränk, Hartkaramelle, Kaugummi oder Mundwasser, vorzugsweise als Getränk, hergerichtet ist.

Was die konkreten Verwendungen der erfindungsgemäßen Zusammensetzungen anbelangt, so sind diese variabel. Einerseits ist ein pharmazeutisch-medizinischer Einsatz der Zusammensetzung zur präventiven bzw. therapeutischen Behandlung von Bluthochdruck möglich. Gleichermaßen können die erfindungsgemäßen Zusammensetzungen auch als Nahrungsmittel oder Nahrungsergänzungsmittel, wie z. B. Getränke, "Energy-Drinks", Kaugummis etc., Hygieneprodukte, wie Mundspüllösungen, und dergleichen verwendet werden.

Gemäß einer besonders bevorzugten Ausführungsform kann es somit vorgesehen sein, dass die Zusammensetzung gemäß der vorliegenden Erfindung zur Verwendung bei der präventiven und/oder therapeutischen Behandlung von arteriellem Bluthochdruck (Hypertonie) vorgesehen ist. Auf dieser Basis kann eine im Wesentlichen nebenwirkungsfreie Senkung des Blutdrucks auf Basis von natürlichen Wirkstoffen erzielt werden, wie zuvor im Detail ausgeführt.

Darüber hinaus kann es auch vorgesehen sein, dass die erfindungsgemäße Zusammensetzung zur Steigerung des Energielevels und/oder der Konzentration gemäss den Ansprüche verwendet wird. Insbesondere können auf Basis der erfindungsgemäßen Zusammensetzungen sogenannte "Energy-Produkte" bereitgestellt werden, z. B. auf Basis von Getränken, Bonbons, Riegeln oder Kaugummis. Besonders bevorzugt ist im Rahmen der vorliegenden Erfindung die Bereitstellung von "Energy-Getränken" mit anregender bzw. stimulierender und gleichzeitig blutdrucksenkender Wirkung, wie nachfolgend auch im Rahmen der Ausführungsbeispiele geschildert.

Insgesamt wird im Rahmen der vorliegenden Erfindung somit eine vielseitig einsetzbare Zusammensetzung bereitgestellt, welche sowohl im medizinischen Bereich als Blutdrucksenker als auch im "Lifestyle-Bereich" als Nahrungsmittel bzw. Nahrungsergänzungsmittel oder Hygiene- bzw. Drogerieprodukt verwendet werden kann und die eigentlich gegensätzlichen Wirkungen, nämlich eine blutdrucksenkende Wirkung mit einer anregenden bzw. stimulierenden Wirkung, kombiniert.

Des Weiteren betrifft die vorliegende Erfindung - gemäß einem **zweiten** Aspekt der vorliegenden Erfindung - die Verwendung einer Zusammensetzung, wie sie zuvor geschildert wurde, als Nahrungsmittel, Nahrungsergänzungsmittel und/oder Hygieneartikel mit konzentrationsfördernder und/oder anregender Wirkung, zur gleichzeitigen Senkung des arteriellen Blutdrucks.

Bei Verwendung der erfindungsgemäßen Zusammensetzung als Nahrungsmittel, Nahrungsergänzungsmittel und/oder Hygieneartikel mit konzentrationsfördernder und/oder anregender Wirkung ist es im Rahmen der vorliegenden Erfindung bevorzugt, wenn die Zusammensetzung über die Mundhöhle appliziert wird, d.h. durch orale Aufnahme für den Verzehr oder durch topische Applikation, z.B. auf Basis einer Mundspüllösung. Dabei wird - wie oben bereits im Detail ausgeführt - zunächst das in den Zusammensetzungen enthaltene Nitrat durch nitratreduzierende Bakterien der Mundhöhle und Mundflora bzw. im Magen-/Darmtrakt zu Nitrit reduziert, welches wiederum in den Blutkreislauf gelangt und dort zu dem blutdrucksenkenden Stickstoffmonoxid weiterführend reduziert wird.

Für weitergehende Einzelheiten zu der erfindungsgemäßen Verwendung kann auf die vorangehenden Ausführungen zu der erfindungsgemäßen Zusammensetzung verwiesen werden, welche auch im Hinblick auf die erfindungsgemäße Verwendung entsprechend gelten.

Bevorzugte Ausführungsformen der vorliegenden Erfindung werden nachfolgend auf Basis der Ausführungsbeispiele geschildert.

### AUSFÜHRUNGSBEISPIELE:

### 1. Herstellungsbeispiele

Um die Wirksamkeit von erfindungsgemäßen Zusammensetzungen insbesondere im Hinblick auf die blutdrucksenkende Wirkung zu untersuchen, wurden zunächst verschiedene pflanzliche Nitratquellen (Saft von roter, weißer und gelber Bete mit jeweils 7 mmol Nitrat (entspricht einer gewichtsbezogenen Nitratmenge von 434 mg pro Applikationseinheit von 150 bis 250 ml)) als solche im Hinblick auf ihre blutdrucksenkende Wirkung untersucht.

Darüber hinaus wurden erfindungsgemäße Zusammensetzungen A, B und C bereitgestellt, welche eine pflanzliche Nitratquelle in flüssiger Form auf Basis von *Beta vulgaris* in Kombination mit weiteren Inhaltsstoffen, nämlich Koffein, Vitamin C, Magnesium und Sulforaphan enthielten. Der nachfolgenden Tabelle sind die Rezepturen für die bereitgestellten Zusammensetzungen A bis C zu entnehmen:

**Tabelle 1: Erfindungsgemäße Rezepturen**

| Inhaltsstoff | Zusammensetzung A | Zusammensetzung B | Zusammensetzung C |
|---|---|---|---|
| Nitratquelle | Weiße Bete-Saft (150 ml) | Gelbe Bete-Saft (100 ml) | Rote Bete-Saft (250 ml) |
| Nitratgehalt | 434 mg (7 mmol) | 434 mg (7 mmol) | 434 mg (7 mmol) |
| Stimulans | Koffein (100 mg) | Koffein (100 mg) | Koffein (100 mg) |
| Vitamin | Vitamin C (250 mg) | Vitamin C (250 mg) | Vitamin C (250 mg) |
| Mineralstoff | Magnesium (150 mg) | Magnesium (150 mg) | Magnesium (150 mg) |
| Indirektes Antioxidans | Sulforaphan (25 mg) | Sulforaphan (25 mg) | Sulforaphan (25 mg) |
| Verhältnis Nitrat/Koffein | 4,34 : 1 | 4,34 : 1 | 4,34 : 1 |

Die oben aufgeführten Zusammensetzungen gemäß Tabelle 1 wurden mit dem Fachmann an sich bekannten Methoden hergestellt. Dazu wurden der flüssigen Nitratquelle auf Basis von Bete-Saft die weiteren Inhaltsstoffe, d. h. Vitamin C, Magnesium, Sulforaphan und Koffein, in den in Tabelle 1 aufgeführten Mengen zugefügt.

Die Bestimmung der Nitratmenge bzw. des Nitratgehalts in den Nitratquellen erfolgte ebenfalls mit dem Fachmann an sich geläufigen Methoden, nämlich durch Hochleistungsflüssigkeitschromatographie (HPLC, eingesetztes Gerät: ENO-20, Fa. Eicom, San Diego, USA).

### 2. Blutdrucksenkende Wirkung von Weißer und Gelber Bete

Bevor der Einfluss der erfindungsgemäßen Zusammensetzungen A bis C gemäß Tabelle 1 mit Nitrat, Koffein und den weiteren Inhaltsstoffen auf den Blutdruck analysiert wurde, wurde zunächst festgestellt, welchen Einfluss die in den erfindungsgemäßen Zusammensetzungen eingesetzten Nitratquellen auf Basis von Bete-Säften aus Roter, Gelber und Weißer Bete ohne zusätzliche Wirkstoffe, insbesondere ohne Koffein, auf den Blutdruck nehmen. Zudem wurde in diesem Zusammenhang auch untersucht, ob Säfte aus Gelber und Weißer Bete überhaupt eine blutdrucksenkende Wirkung besitzen. Bislang wurde im Stand der Technik nämlich lediglich Rote Bete im Zusammenhang mit einer blutdrucksenkenden Wirkung beschrieben.

Um die blutdrucksenkende Wirkung von Saft aus Weißer, Gelber und Roter Bete zu vergleichen, wurden die Säfte mit jeweils 7 mmol bzw. 434 mg Nitrat pro Portion bzw. Applikationseinheit (150 bis 250 ml) bereitgestellt. Die Bestimmung der Nitratkonzentration erfolgte, wie oben beschrieben, mittels HPLC.

### Studiendesign

Zur Untersuchung der Wirkung wurde ein Probandenkollektiv auf Basis von 30 männlichen Probanden im Alter von 35 bis 55 Jahren herangezogen. Alle Probanden waren leicht übergewichtig mit einem BMI zwischen 27 bis 30, Raucher und wiesen einen Blutdruck im oberen normalen oder hohen Bereich auf. Die 30 Probanden wurden auf drei Probandengruppen von jeweils zehn Probanden aufgeteilt, wobei jede Probengruppe einen der Säfte testete (Gruppe 1: Rote Bete, Gruppe 2: Weiße Bete, Gruppe 3: Gelbe Bete). Zu Vergleichszwecken wurden zudem weitere fünf Probanden herangezogen, welche einen niedrigen systolischen Blutdruck von maximal 110 mmHg hatten. Probanden dieser Vergleichsgruppe erhielten Rote Bete-Saft.

Im Rahmen der Studie erhielten die Probanden eine Applikationseinheit des jeweiligen Bete-Saftes mit 7 mmol bzw. 434 mg Nitrat zum Verzehr. Um die blutdrucksenkende Wirkung zu ermitteln, wurde der systolische Blutdruck unmittelbar vor Verzehr der Bete-Säfte gemessen. Eine zweite Messung erfolgte zwei Stunden (120 Minuten) nach Verzehr.

### Ergebnisse

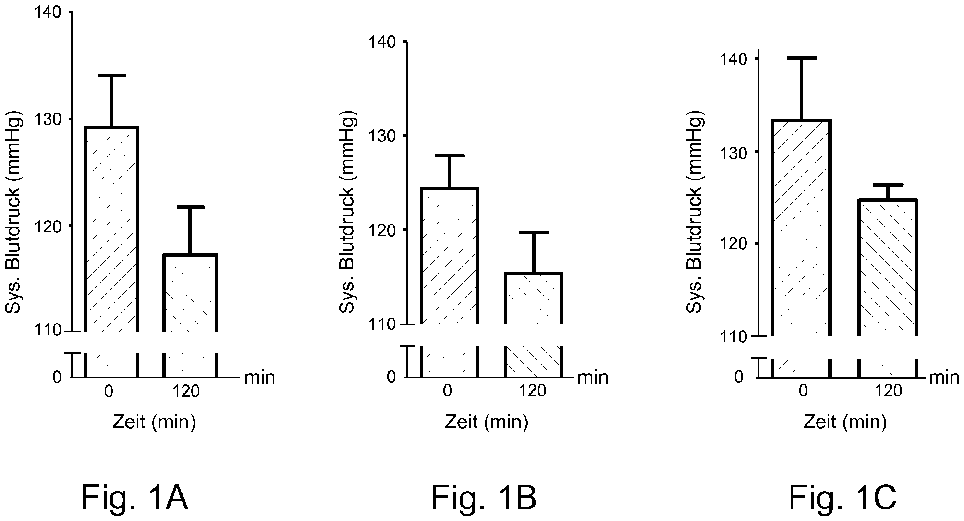

Die im Rahmen der Blutdruckmessungen vor und zwei Stunden nach Verzehr der Säfte, jeweils über alle Probanden der Gruppe gemittelten erhaltenen systolischen Blutdruckwerte sind in Fig. 1 dargestellt (Fig. 1A: Rote Bete, Figur 1B: Weiße Bete, Figur 1C: Gelbe Bete).

Bei Gruppe 1 (Fig. 1A) lag der systolische Blutdruckwert der Probanden unmittelbar vor Einnahme des Saftes bei circa 129 mmHg. Zwei Stunden nach Verzehr lag der Blutdruck der Probanden im Schnitt deutlich unter 120 mmHg, d.h. es konnte eine Blutdrucksenkung von mehr als 10 mmHg erzielt werden. Bei Gruppe 2 (Fig. 1B), welche Weiße Bete-Saft erhalten hatte, lag der durchschnittliche systolische Blutdruck der Probanden vor Einnahme des Saftes bei circa 124 mmHg, d.h. im oberen normalen, nicht optimalen Bereich. Zwei Stunden nach Verzehr des Saftes betrug der durchschnittliche systolische Blutdruck bei den Probanden nur noch circa 115 mmHg und war somit in den optimalen Bereich gesunken. Gruppe 3 (Fig. 1C) erhielt Gelbe Bete-Saft. Der durchschnittliche systolische Blutdruck vor Verzehr des Saftes lag bei den Probanden dieser Gruppe im Schnitt bei circa 133 mmHg. Zwei Stunden nach Verzehr war der Blutdruck auf durchschnittliche Werte von ungefähr 123 mmHg gesunken.

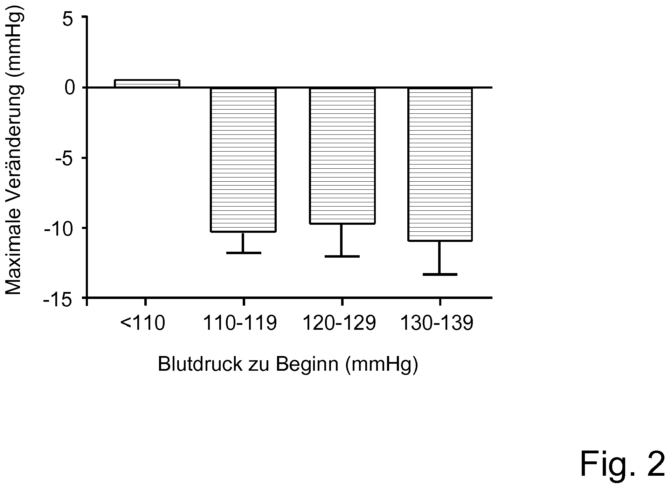

Fig. 2 zeigt zudem die Wirkung von Bete-Saft in Abhängigkeit vom Ausgangsblutdruck auf Basis einer Gegenüberstellung der maximalen Veränderung des Blutdrucks in der Vergleichsgruppe mit niedrigem Blutdruck und den übrigen Probanden mit normalem bis hohen Blutdruck. Dabei ist ersichtlich, dass bei Probanden mit niedrigem Blutdruck von maximal 110 mmHg keine weitere Senkung des Blutdrucks eingetreten ist. Bei den übrigen Probanden hingegen konnte der Blutdruck durchschnittlich um circa 10 mmHg gesenkt werden.

Alle getesteten Bete-Säfte mit einem Nitratgehalt von 434 mg pro Applikationseinheit bzw. pro Portion führten bei den Probanden zu einer deutlichen Senkung des systolischen Blutdrucks. In diesem Zusammenhang hat sich auch gezeigt, dass die erfindungsgemäß bevorzugten Nitratquellen auf Basis von Gelber und Weißer Bete gleichermaßen zur Erzielung einer Blutdrucksenkung geeignet sind. Zudem hat sich im Rahmen der Studie gezeigt, dass die blutdrucksenkende Wirkung nur eintritt, wenn der Blutdruck im normalen oder hohen Bereich liegt. Ein niedriger Blutdruck hingegen wird nicht weiterführend gesenkt.

### 3. Blutdrucksenkende Wirkung der erfindungsgemäßen Zusammensetzungen

Weiterhin wurde mittels Probandenstudien auch die Wirkung der in Tabelle 1 aufgeführten, erfindungsgemäßen Zusammensetzungen, welche neben der Nitratquelle auf Basis von Bete-Saft auch Koffein als stimulierende Substanz und darüber hinaus Vitamin C, Magnesium und Sulforaphan enthielten, untersucht. In diesem Zusammenhang sollte insbesondere analysiert werden, ob die Zugabe von Koffein, welches eine zu der Wirkung von Nitrat konträre, da anregende Wirkung besitzt, die blutdrucksenkende Wirkung der Nitratquelle auf Basis von Bete-Saft aufgehoben wird.

### Studiendesign

Zur Untersuchung der Wirkung wurde ein Probandenkollektiv auf Basis von 12 männlichen Probanden im Alter von 35 bis 55 Jahren herangezogen. Alle Probanden waren leicht übergewichtig mit einem BMI zwischen 27 bis 30, Raucher und litten unter arterieller Hypertonie. Die 12 Probanden wurden auf drei Probandengruppen von jeweils vier Probanden aufgeteilt, wobei jede Probengruppe eine der Zusammensetzungen A bis C testete (Gruppe 1: Zusammensetzung A, Gruppe 2: Zusammensetzung B, Gruppe 3: Zusammensetzung C).

Im Rahmen der Studie erhielten die Probanden eine Applikationseinheit der jeweiligen Zusammensetzung mit 7 mmol bzw. 434 mg Nitrat zum Verzehr. Um die blutdrucksenkende Wirkung zu ermitteln, wurde der systolische Blutdruck unmittelbar vor Verzehr der jeweiligen Zusammensetzung gemessen. Eine zweite Messung erfolgte zwei Stunden (120 Minuten) nach Verzehr. Darüber hinaus wurde die Konzentration von Nitrat und Nitrit im Blutplasma der Probanden vor Verzehr der erfindungsgemäßen Zusammensetzungen und zwei Stunden nach Gabe der Zusammensetzung untersucht. Die Messung der Nitrat- und Nitritkonzentration im Plasma der Probanden erfolgte ebenfalls mittels HPLC.

### Ergebnisse

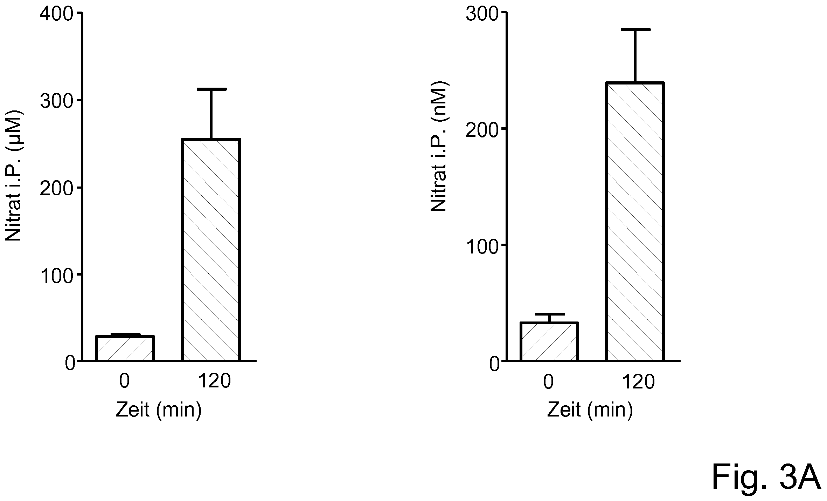

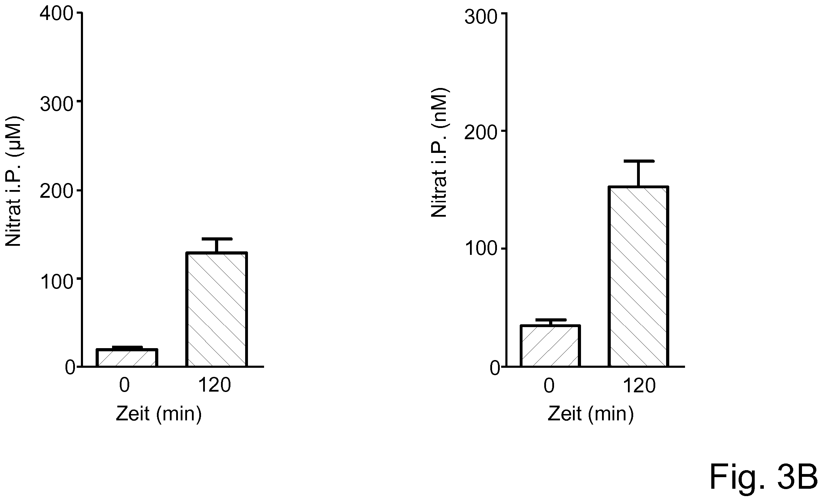

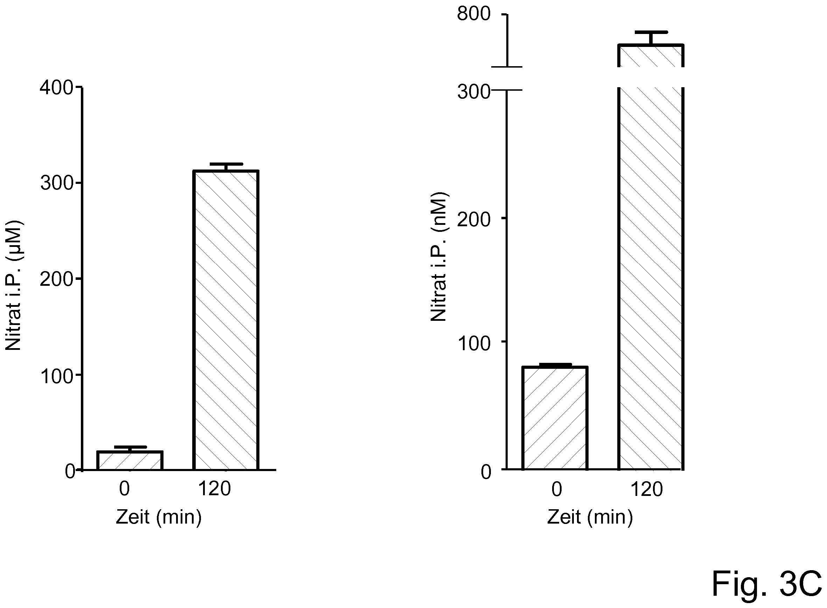

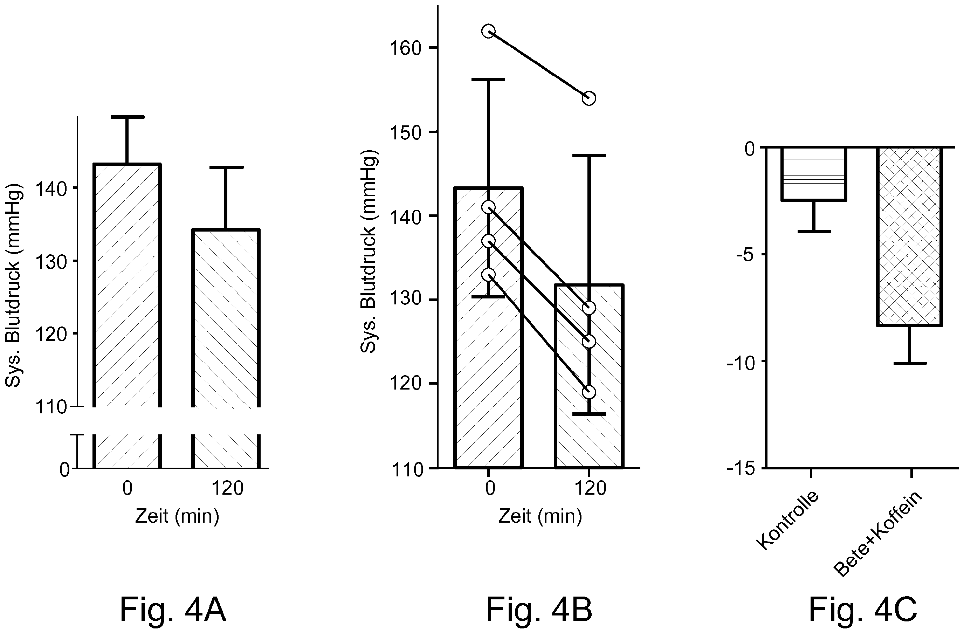

Die Ergebnisse der Blutuntersuchung bezüglich der durchschnittlichen Nitrat- und Nitritkonzentration im Plasma der Probanden sind in Fig. 3A (Gruppe 3, Zusammensetzung C, Rote Bete), Fig. 3B (Gruppe 1, Zusammensetzung A, Weiße Bete) und Fig. 3C (Gruppe 2, Zusammensetzung B, Gelbe Bete) enthalten. In Fig. 4 sind die konkreten Effekte von Zusammensetzung A (Gruppe 1, Weiße Bete) auf den systolischen Blutdruck dargestellt. Fig. 4A zeigt dabei die im Rahmen der Blutdruckmessungen vor Verzehr der Zusammensetzung und zwei Stunden nach Verzehr erhaltenen, gemittelten Blutdruckwerte. Fig. 4B zeigt die Ergebnisse einer weiterführenden Auswertung, welche den Zusammenhang zwischen basalem Blutdruck (Ausgangsblutdruck) und erreichten Minimalwerten des Blutdrucks erfasst. Schließlich ist in Fig. 4C die durchschnittliche bzw. gemittelte Blutdrucksenkung bei den Probanden, die Zusammensetzung A erhalten hatten, gegenüber einer (Negativ-)Kontrollgruppe dargestellt.

Zusammensetzung C mit Rote Bete-Saft hat, wie aus Fig. 3A ersichtlich, zwei Stunden nach Verzehr zu einem signifikanten Anstieg sowohl der Nitrat- als auch der Nitritkonzentration im Plasma der Probanden geführt. Auch Zusammensetzung A mit Weiße Bete-Saft als Nitratquelle führt zu einem Anstieg des Nitrat- und Nitritlevels im Blut (vgl. Fig. 3B). Schließlich kann auch mit Zusammensetzung B auf Basis von Gelbe Bete-Saft ein deutlicher Anstieg der Nitrat- und Nitritkonzentration im Plasma der Probanden erreicht werden (vgl. Fig. 3C). Die erfindungsgemäßen Zusammensetzungen bieten somit eine geeignete Basis, um tatsächlich die Konzentration von Stickstoffmonoxid, welches als Vasodilatator eine gefäßerweiternde und damit blutdrucksenkende Wirkung besitzt, im Blut zu erhöhen.

Um die blutdrucksenkende Wirkung der erfindungsgemäßen Zusammensetzungen weiterführend zu untersuchen, wurde Zusammensetzung A auf Basis von Weiße Bete-Saft herangezogen:
Fig. 4A zeigt die im Rahmen der Blutdruckmessungen vor und zwei Stunden nach Verzehr der Zusammensetzung A erhaltenen und jeweils über alle Probanden der Gruppe gemittelten systolischen Blutdruckwerte. Vor Verzehr der Zusammensetzungen wiesen die Probanden im Schnitt einen systolischen Blutdruck von circa 143 mmHg auf, d.h. Werte im Bereich der arteriellen Hypertonie. Zwei Stunden nach Verzehr von Zusammensetzung A waren die Werte auf durchschnittlich circa 132 mmHg gesunken, d.h. der Blutdruck konnte auf Werte im hohen Normalbereich gesenkt werden.

In Fig. 4B sind die basalen Blutdruckwerte einzelner Probanden und die bei den gleichen Probanden gemessenen Minimalwerte korreliert. Aus dieser Korrelation geht zum einen hervor, dass bei jedem einzelnen Probanden eine Senkung des Blutdrucks auf Basis der erfindungsgemäßen Zusammensetzung erzielt werden konnte. Zum anderen ist gleichermaßen ersichtlich, dass der jeweils gemessene minimale Blutdruck von dem basalen Blutdruck abhängt. Insgesamt kann durch die erfindungsgemäßen Zusammensetzungen somit der systolische Blutdruck zuverlässig um circa 10 bis 15 mmHg gesenkt werden. Ein zu starkes Abfallen des Blutdrucks, was mit unerwünschten Symptomen, wie Schlappheit, Schwindel etc., einhergehen kann, tritt hingegen nicht ein. Fig. 4C stellt zudem die Ergebnisse der durchschnittlichen Blutdrucksenkung (Differenz syst. Blutdruck [in mmHg]) in einer Kontrollgruppe, welche keine blutdrucksenkende Zusammensetzung erhalten hatte, und den Probanden, die Zusammensetzung A erhalten hatten, gegenüber. In der Kontrollgruppe wurde lediglich eine durchschnittliche Blutdrucksenkung von 2,5 mmHg gemessen, wohingegen Zusammensetzung A zu einer durchschnittlichen Blutdrucksenkung von circa 8 mmHg geführt hatte.

### 4. Zusammenfassung

Auf Basis der obigen Ausführungen ist ersichtlich, dass im Rahmen der vorliegenden Erfindung eine blutdrucksenkende Zusammensetzung bereitgestellt wird, welche auf gutverträglichen, natürlichen Inhaltsstoffen, nämlich pflanzlichen Nitratquellen, Antioxidantien und Mineralstoffen, gemäss der Ansprüchen, basiert und zu einer signifikanten Reduzierung des systolischen Blutdrucks führt. Vor dem Hintergrund, dass die Zusammensetzung auf natürlichen Inhaltsstoffen basiert, ist sie im Wesentlichen frei von unerwünschten Nebenwirkungen, welche im Zusammenhang mit einer medikamentösen Therapie von Bluthochdruck auftreten können.

Besonders überraschend führt auch der kombinierte Einsatz einer anregenden bzw. konzentrationsfördernden Substanz auf Basis von Koffein nicht zu einer Aufhebung der blutdrucksenkenden Wirkung.

Im Ergebnis wird somit eine nebenwirkungsfreie Zusammensetzung bereitgestellt, welche zur nicht-medikamentösen Blutdrucksenkung geeignet ist und darüber hinaus weiterführende physiologische Eigenschaften aufweist, insbesondere eine konzentrationsfördernde und anregende Wirkung, welche noch durch antioxidative Wirkungen und für den Körper lebensnotwendige Mineralstoffe ergänzt wird.

Vorteilhaft an der vorliegenden Erfindung bzw. den erfindungsgemäßen Zusammensetzungen ist zudem, dass die blutdrucksenkende Wirkung nur bei einem über dem Normbereich, d. h. einem zu hohen Blutdruck, oder einem im oberen Normbereich, d. h. im Bereich zwischen 120 und 130 mmHg, liegenden systolischen Blutdrucks auftritt. Bei niedrigem Blutdruck hingegen kommt es zu keiner weiteren Reduktion des Blutdrucks, d. h. die Gefahr einer Hypotonie, besteht nicht. Somit ist die erfindungsgemäße Zusammensetzung auch für Personen mit niedrigem oder normalem Blutdruck geeignet, so dass diese von den weiteren vorteilhaften Eigenschaften der erfindungsgemäßen Zusammensetzung, insbesondere der konzentrationsfördernden und anregenden Wirkung sowie den antioxidativen Effekten und der Supplementation mit Mineralstoffen profitieren können.

## Patentansprüche

1. Zusammensetzung mit konzentrationsfördernder und/oder anregender Wirkung zur Verwendung bei der präventiven und/oder therapeutischen Behandlung von arteriellem Bluthochdruck (Hypertonie), wobei die Zusammensetzung
(a) als Komponente (a) mindestens eine Nitratquelle;
(b) als Komponente (b) Koffein; sowie
Sulforaphan als Substanz mit indirekter antioxidativer Wirkung
enthält, und
wobei die Komponente (a) und/oder die Nitratquelle Nitrat in einer Menge, bezogen auf die gewichtsbezogene Menge an NO₃ und/oder NO₃⁻ pro Portion und/oder Applikationseinheit der Zusammensetzung, im Bereich 10 bis 5.000 mg enthält,
wobei die Zusammensetzung die Komponente (b), bezogen auf eine Portion und/oder Applikationseinheit der Zusammensetzung, in einer Menge im Bereich von 10 bis 500 mg enthält; und
wobei die Zusammensetzung Nitrat und die Komponente (b) in einem gewichtsbezogenen Mengenverhältnis von [gewichtsbezogene Menge NO₃]: [gewichtsbezogene Menge Komponente (b)] im Bereich von 1:2 bis 20 : 1 enthält.

2. Zusammensetzung zur Verwendung nach Anspruch 1,
wobei die Komponente (a) und/oder die Nitratquelle Nitrat in einer Menge, bezogen auf die gewichtsbezogene Menge an NO₃ und/oder NO₃⁻ pro Portion und/oder Applikationseinheit der Zusammensetzung, im Bereich von 100 bis 2.500 mg, bevorzugt 200 bis 1.000 mg,-besonders bevorzugt 300 bis 700 mg, enthält; und/oder
wobei die Komponente (a) und/ oder die mindestens eine Nitratquelle ausgewählt ist aus pflanzlichen Nitratquellen und/oder Salzen und/oder Estern, insbesondere Salzen, der Salpetersäure und/oder deren Mischungen, vorzugsweise pflanzlichen Nitratquellen.

3. Zusammensetzung zur Verwendung nach Anspruch 2,
wobei die pflanzliche Nitratquelle ausgewählt ist aus Pflanzen der Gruppe von *Spinacia oleracea* (Gemüsespinat), *Raphanus* (Rettich), *Beta vulgaris* (Gemeine Rübe), *Brassica rapa* (Speiserübe), *Diplotaxis tenuifolia* (Rucola), *Lepidium* (Kresse), *Valerianella* (Feldsalat), vorzugsweise *Beta vulgaris* (Gemeine Rübe), und/ oder
wobei die pflanzliche Nitratquelle aus der gelben, roten und/oder weißen Kulturform, bevorzugt aus der gelben und/oder weißen Kulturform, von *Beta vulgaris* (Gemeine Rübe) gewonnen ist; und/oder
wobei die Zusammensetzung die pflanzliche Nitratquelle in Form von flüssigen Pflanzenbestandteilen, insbesondere Pflanzenextrakt und/oder Pflanzensaft, und/oder in Form von festen Pflanzenbestandteilen, insbesondere Blättern, Wurzeln und/oder Speicherorganen in vorzugsweise zermahlener Form, enthält, besonders bevorzugt in Form von Pflanzenextrakt und/oder Pflanzensaft.

4. Zusammensetzung zur Verwendung nach Anspruch 2, wobei die Komponente (a) und/oder die
Nitratquelle in Form von Salzen und/oder Estern der Salpetersäure, insbesondere Salzen der Salpetersäure, ausgewählt ist aus den Alkali- und Erdalkalisalzen der Salpetersäure, insbesondere Kaliumnitrat, Natriumnitrat, Bariumnitrat, Calciumnitrat, Magnesiumnitrat und/oder deren Mischungen, bevorzugt Kaliumnitrat und/oder Natriumnitrat.

5. Zusammensetzung zur Verwendung nach einem der vorangehenden Ansprüche,
wobei die Zusammensetzung als Komponente (b) Koffein enthält und/oder wobei die Zusammensetzung die Komponente (b), bezogen auf eine Portion und/oder Applikationseinheit der Zusammensetzung, in einer Menge im Bereich von 50 bis 250 mg, besonders bevorzugt 80 bis 150 mg, enthält; und/oder
wobei die Zusammensetzung Nitrat und die Komponente (b) in einem gewichtsbezogenen Mengenverhältnis von [gewichtsbezogene Menge NO₃] : [gewichtsbezogene Menge Komponente (b)] im Bereich von 1 : 1 bis 10 : 1, enthält.

6. Zusammensetzung zur Verwendung nach einem der vorangehenden Ansprüche, wobei die
Zusammensetzung als Komponente (c) mindestens einen Mineralstoff enthält, insbesondere wobei die Komponente (c) ausgewählt ist aus der Gruppe von Calcium, Chlor, Kalium, Magnesium, Natrium, Phosphor, Schwefel, Eisen, lod, Kupfer, Mangan, Molybdän, Selen, Zink und/oder deren Mischungen, vorzugsweise Magnesium,
insbesondere wobei die Zusammensetzung die Komponente (c), bezogen auf eine Portion und/oder Applikationseinheit der Zusammensetzung, in einer Menge im Bereich von 1 bis 1.000 mg, insbesondere 5 bis 500 mg, vorzugsweise 10 bis 250 mg, bevorzugt 50 bis 200 mg, enthält.

7. Zusammensetzung zur Verwendung nach einem der vorangehenden Ansprüche, wobei die
Zusammensetzung Vitamin C, bezogen auf eine Portion und/oder Applikationseinheit der Zusammensetzung, in einer Menge im Bereich von 1 bis 5.000 mg, insbesondere 5 bis 2.500 mg, vorzugsweise 10 bis 1.500 mg, bevorzugt 50 bis 1.000 mg, besonders bevorzugt 100 bis 500 mg, noch weiter bevorzugt 200 bis 400 mg, enthält.

8. Zusammensetzung zur Verwendung nach einem der vorangehenden Ansprüche, wobei die
Zusammensetzung die Komponente (e) und/oder Sulforaphan, bezogen auf eine Portion und/oder Applikationseinheit der Zusammensetzung, in einer Menge im Bereich von 0,01 bis 500 mg, insbesondere 0,1 bis 250 mg, vorzugsweise 1 bis 100 mg, bevorzugt 5 bis 50 mg, besonders bevorzugt 10 bis 30 mg, enthält.

9. Zusammensetzung zur Verwendung nach einem der vorangehenden Ansprüche, wobei die
Zusammensetzung als Hilfsstoff Kaugummigrundmasse enthält,
insbesondere wobei die Kaugummigrundmasse natürliche Polymere, insbesondere Kautschuk, vorzugsweise in Form von Latex, Crepe oder Sheets, Polyole sowie deren Mischungen, enthält und/oder insbesondere wobei die Kaugummigrundmasse synthetische Polymere, insbesondere synthetische Thermoplaste, vorzugsweise Polyvinylether, insbesondere Poly(ethylvinylether) und/oder Poly(isobutylvinylether), Polyvinylacetate, Vinylacetate, Polyisobutene und/oder deren Mischungen, enthält.

10. Zusammensetzung zur Verwendung nach einem der vorangehenden Ansprüche, wobei die
Zusammensetzung als Hilfsstoff mindestens ein festes Träger- und/oder Matrixmaterial enthält,
insbesondere wobei das Träger- und/oder Matrixmaterial ausgewählt ist aus Zuckern und/oder Zuckeraustauschstoffen, vorzugsweise aus der Gruppe von Saccharose, Glucose, Dextrose, Fructose, Mannit, Xylit, Sorbit, Isomalt, Maltitsirup, Lactit, Leucrose, Fructooligosacchariden, Glucanen, Polyglucose und/oder deren Mischungen.

11. Zusammensetzung zur Verwendung nach einem der vorangehenden Ansprüche, wobei die
Zusammensetzung mindestens ein flüssiges Trägermaterial enthält,
insbesondere wobei das Trägermaterial polare und/oder apolare Lösungsmitteln enthält, vorzugsweise Wasser, Ethanol, Glycerin, hydriertes Rizinusöl (*hydrogenated castor oil*), Polyethylenglykol, Polypropylenglycol und/oder deren Mischungen.

12. Zusammensetzung zur Verwendung nach einem der vorangehenden Ansprüche, wobei die
Zusammensetzung mindestens einen weiteren Inhaltsstoff enthält,
insbesondere wobei der mindestens eine weitere Inhaltsstoff ausgewählt ist aus der Gruppe von Zusatz- und/oder Hilfsstoffen, vorzugsweise Binde-, Netz-, Stabilisierungs-, Färbe-, Puffer-, Riech-, Geschmacks-, Süßungs-, Aroma-, Verarbeitungshilfs- und Konservierungsstoffen und -mitteln und/oder deren Mischungen.

13. Zusammensetzung zur Verwendung nach einem der vorangehenden Ansprüche,
wobei die Zusammensetzung für den Verzehr und/oder für die topische Applikation, insbesondere in der Mundhöhle, hergerichtet ist; und/oder
wobei die Zusammensetzung in fester, flüssiger, viskoser (gelförmiger), halbfester, aerosolförmiger oder pastöser Form, insbesondere flüssiger oder fester Form, hergerichtet ist; und/oder
wobei die Zusammensetzung als Getränk, Riegel, Gebäckstück, Hartkaramelle, Kaugummi, Zahnpasta und/oder Mundwasser (Mundspüllösung), insbesondere als Getränk, Hartkaramelle, Kaugummi oder Mundwasser, vorzugsweise als Getränk, hergerichtet ist.

## Claims

1. A composition with a concentration-promoting and/or stimulating effect for use in the preventive and/or therapeutic treatment of arterial hypertension (hypertension), said composition containing
(a) as component (a), at least one nitrate source;
(b) as component (b), caffeine; and
sulforaphane as a substance having indirect antioxidant activity and
wherein component (a) and/or the nitrate source contains nitrate in an amount, based on the amount of NO₃ and/or NO₃⁻ by weight per serving and/or application unit of the composition, in the range of 10 to 5,000 mg,
wherein the composition contains component (b) in an amount ranging from 10 to 500 mg based on a serving and/or unit of application of the composition; and
wherein the composition comprises nitrate and component (b) in a weight based amount ratio of [weight based amount NO₃] : [weight based amount component (b)] ranging from 1 : 2 to 20 : 1.

2. A composition for use according to claim 1,
wherein component (a) and/or the nitrate source contains nitrate in an amount, based on the weight-related amount of NO₃ and/or NO₃⁻ per portion and/or application unit of the composition, in the range of 100 to 2,500 mg, preferably 200 to 1,000 mg, particularly preferably 300 to 700 mg; and/or
wherein component (a) and/or the at least one nitrate source is selected from vegetable nitrate sources and/or salts and/or esters, in particular salts, of nitric acid and/or mixtures thereof, preferably vegetable nitrate sources.

3. A composition for use according to claim 2,
wherein the vegetable nitrate source is selected from plants of the group of Spinacia oleracea (vegetable spinach), Raphanus (radish), Beta vulgaris (common turnip), Brassica rapa (turnip), Diplotaxis tenuifolia (rocket), Lepidium (cress), Valerianella (lamb's lettuce), preferably Beta vulgaris (common turnip), and/or
wherein the vegetable nitrate source is derived from the yellow, red and/or white cultivated form, preferably the yellow and/or white cultivated form, of Beta vulgaris (common turnip); and/or
wherein the composition contains the plant nitrate source in the form of liquid plant components, in particular plant extract and/or plant juice, and/or in the form of solid plant components, in particular leaves, roots and/or storage organs in preferably ground form, particularly preferably in the form of plant extract and/or plant juice.

4. Composition for use according to claim 2, wherein component (a) and/or the nitrate source in the form of salts and/or esters of nitric acid, in particular salts of nitric acid, is selected from the alkali metal and alkaline earth metal salts of nitric acid, in particular potassium nitrate, sodium nitrate, barium nitrate, calcium nitrate, magnesium nitrate and/or mixtures thereof, preferably potassium nitrate and/or sodium nitrate.

5. Composition for use according to any one of the preceding claims,
wherein the composition contains caffeine as component (b) and/or wherein the composition contains component (b) in an amount in the range of 50 to 250 mg, particularly preferably 80 to 150 mg, based on a portion and/or application unit of the composition; and/or
wherein the composition contains nitrate and component (b) in a weight-based amount ratio of [weight-based amount NO3] : [weight-based amount component (b)] in the range of 1 : 1 to 10 : 1.

6. Composition for use according to one of the preceding claims, wherein the composition contains as component (c) at least one mineral, in particular wherein component (c) is selected from the group of calcium, chlorine, potassium, magnesium, sodium, phosphorus, sulfur, iron, iodine, copper, manganese, molybdenum, selenium, zinc and/or mixtures thereof, preferably magnesium,
in particular wherein the composition contains component (c), based on a portion and/or application unit of the composition, in an amount in the range of 1 to 1,000 mg, in particular 5 to 500 mg, preferably 10 to 250 mg, preferably 50 to 200 mg.

7. A composition for use according to any one of the preceding claims, wherein the composition contains vitamin C, based on a serving and/or application unit of the composition, in an amount in the range of 1 to 5,000 mg, in particular 5 to 2,500 mg, preferably 10 to 1,500 mg, particularly preferably 50 to 1,000 mg, more preferably 100 to 500 mg, still more preferably 200 to 400 mg.

8. A composition for use according to any one of the preceding claims, wherein the composition comprises component (e) and/or sulforaphane, based on a serving and/or application unit of the composition, in an amount in the range of 0.01 to 500 mg, in particular 0.1 to 250 mg, preferably 1 to 100 mg, particularly preferably 5 to 50 mg, more preferably 10 to 30 mg.

9. Composition for use according to one of the preceding claims, wherein the composition contains chewing gum base as excipient,
in particular wherein the chewing gum base comprises natural polymers, in particular chewing gum, preferably in the form of latex, crepe or sheets, polyols as well as mixtures thereof, and/or in particular wherein the chewing gum base comprises synthetic polymers, in particular synthetic thermoplastics, preferably polyvinyl ethers, particularly preferably poly(ethyl vinyl ether) and/or poly(isobutyl vinyl ether), polyvinyl acetates, vinyl acetates, polyisobutenes and/or mixtures thereof.

10. Composition according to any one of the preceding claims, wherein the composition comprises as excipient at least one solid carrier and/or matrix material,
in particular wherein the carrier and/or matrix material is selected from sugars and/or sugar substitutes, preferably from the group of sucrose, glucose, dextrose, fructose, man¬nitol, xylitol, sorbitol, isomalt, maltitol syrup, lactitol, leucrose, fructooligosaccharides, glucans, polyglucose and/or mixtures thereof.

11. Composition for use according to any one of the preceding claims, wherein the composition comprises at least one liquid carrier material,
in particular wherein the carrier material comprises polar and/or apolar solvents, preferably water, ethanol, glycerol, hydrogenated castor oil, polyethylene glycol, polypropylene glycol and/or mixtures thereof.

12. A composition for use according to any one of the preceding claims, wherein the composition contains at least one further ingredient,
in particular wherein the at least one further ingredient is selected from the group of additives and/or adjuvants, preferably binding, wetting, stabilizing, coloring, buffering, smelling, flavoring, sweetening, aroma, processing aids and preservatives and agents and/or mixtures thereof.

13. A composition for use according to any one of the preceding claims,
wherein the composition is prepared for consumption and/or for topical application, in particular in the oral cavity; and/or
wherein the composition is prepared in solid, liquid, viscous (gel-like), semi-solid, aerosol or pasty form, in particular liquid or solid form; and/or
wherein the composition is prepared as a beverage, bar, pastry, hard caramel, chewing gum, toothpaste and/or mouthwash, in particular as a beverage, hard caramel, chewing gum or mouthwash, preferably as a beverage.

## Revendications

1. Composition ayant un effet favorisant la concentration et/ou stimulant pour utilisation dans le traitement préventif et/ou thérapeutique de l'hypertension artérielle (hypertension), ladite composition contenant
(a) comme composant (a), au moins une source de nitrate;
(b) comme composant (b), de la caféine; et
du sulforaphane en tant que substance ayant une activité antioxydante indirecte
et
dans laquelle le composant (a) et/ou la source de nitrate contient du nitrate en une quantité, basée sur la quantité de NO₃ et/ou de NO₃⁻ en poids par portion et/ou unité d'application de la composition, comprise entre 10 et 5 000 mg,
dans laquelle la composition contient le composant (b) en une quantité allant de 10 à 500 mg sur la base d'une portion et/ou d'une unité d'application de la composition; et
dans laquelle la composition comprend du nitrate et le composant (b) dans un rapport de quantité en poids de [quantité en poids de NO₃] : [quantité en poids de composant (b)] allant de 1 : 2 à 20 : 1.

2. Composition à utiliser selon la revendication 1,
dans laquelle le composant (a) et/ou la source de nitrate contient du nitrate en une quantité, basée sur la quantité pondérale de NO₃ et/ou de NO₃⁻ par portion et/ou unité d'application de la composition, comprise entre 100 et 2 500 mg, de préférence entre 200 et 1 000 mg, de préférence encore entre 300 et 700 mg; et/ou
dans laquelle le composant (a) et/ou l'au moins une source de nitrate est choisi parmi les sources de nitrate végétal et/ou les sels et/ou les esters, en particulier les sels, de l'acide nitrique et/ou leurs mélanges, de préférence les sources de nitrate végétal.

3. Composition à utiliser selon la revendication 2,
dans laquelle la source de nitrate végétal est choisie parmi les plantes du groupe de Spinacia oleracea (épinard végétal), Raphanus (radis), Beta vulgaris (navet commun), Brassica rapa (navet), Diplotaxis tenuifolia (roquette), Lepidium (cresson), Valerianella (mâche), de préférence Beta vulgaris (navet commun), et/ou
dans laquelle la source de nitrate végétal est dérivée de la forme cultivée jaune, rouge et/ou blanche, de préférence la forme cultivée jaune et/ou blanche, de Beta vulgaris (navet commun); et/ou
dans laquelle la composition contient la source de nitrate végétale sous forme de composants végétaux liquides, en particulier d'extrait végétal et/ou de jus végétal, et/ou sous forme de composants végétaux solides, en particulier de feuilles, de racines et/ou d'organes de stockage, de préférence sous forme broyée, de préférence sous forme d'extrait végétal et/ou de jus végétal.

4. Composition à utiliser selon la revendication 2, dans laquelle le composant (a) et/ou la source de nitrate sous forme de sels et/ou d'esters d'acide nitrique, en particulier les sels d'acide nitrique, est choisi parmi les sels d'acide nitrique des métaux alcalins et des métaux alcalino-terreux, en particulier le nitrate de potassium, le nitrate de sodium, le nitrate de baryum, le nitrate de calcium, le nitrate de magnésium et/ou leurs mélanges, de préférence le nitrate de potassium et/ou le nitrate de sodium.

5. Composition à utiliser selon l'une quelconque des revendications précédentes,
dans laquelle la composition contient de la caféine en tant que composant (b) et/ou dans laquelle la composition contient le composant (b) en une quantité comprise entre 50 et 250 mg, de préférence entre 80 et 150 mg, sur la base d'une portion et/ou d'une unité d'application de la composition; et/ou
dans laquelle la composition contient du nitrate et le composant (b) dans un rapport de quantité basé sur le poids de [quantité basée sur le poids de NO₃] : [quantité basée sur le poids du composant (b)] compris entre 1 : 1 et 10 : 1.

6. Composition à utiliser selon l'une des revendications précédentes, dans laquelle la composition contient comme composant (c) au moins un minéral, en particulier dans laquelle le composant (c) est choisi dans le groupe du calcium, du chlore, du potassium, du magnésium, du sodium, du phosphore, du soufre, du fer, de l'iode, du cuivre, du manganèse, du molybdène, du sélénium, du zinc et/ou de leurs mélanges, de préférence le magnésium,
en particulier dans le cas où la composition contient le composant (c), sur la base d'une portion et/ou d'une unité d'application de la composition, en une quantité comprise entre 1 et 1 000 mg, en particulier entre 5 et 500 mg, de préférence entre 10 et 250 mg, de préférence entre 50 et 200 mg.

7. Composition à utiliser selon l'une quelconque des revendications précédentes, dans laquelle la composition contient de la vitamine C, sur la base d'une portion et/ou d'une unité d'application de la composition, en une quantité comprise entre 1 et 5 000 mg, en particulier entre 5 et 2 500 mg, de préférence entre 10 et 1 500 mg, de manière particulièrement préférentielle entre 50 et 1 000 mg, plus préférentiellement entre 100 et 500 mg, et de manière encore plus préférentielle entre 200 et 400 mg.

8. Composition à utiliser selon l'une quelconque des revendications précédentes, dans laquelle la composition comprend le composant (e) et/ou le sulforaphane, sur la base d'une portion et/ou d'une unité d'application de la composition, en une quantité comprise entre 0,01 et 500 mg, en particulier entre 0,1 et 250 mg, de préférence entre 1 et 100 mg, de préférence encore entre 5 et 50 mg, de préférence encore entre 10 et 30 mg.

9. Composition à utiliser selon l'une des revendications précédentes, dans laquelle la composition contient une base de chewing-gum comme excipient,
en particulier dans laquelle la base de chewing-gum comprend des polymères naturels, en particulier de la gomme à mâcher, de préférence sous forme de latex, de crêpe ou de feuilles, des polyols ainsi que leurs mélanges, et/ou en particulier dans laquelle la base de chewing-gum comprend des polymères synthétiques, en particulier des thermoplastiques synthétiques, de préférence des éthers de polyvinyle, de préférence des poly(éthyl vinyle éther) et/ou des poly(isobutyl vinyle éther), des acétates de polyvinyle, des acétates de vinyle, des polyisobutènes et/ou des mélanges de ces derniers.

10. Composition selon l'une quelconque des revendications précédentes, dans laquelle la composition comprend comme excipient au moins un support solide et/ou une matière matricielle,
en particulier dans laquelle le support et/ou la matrice sont choisis parmi les sucres et/ou les substituts de sucre, de préférence dans le groupe du saccharose, du glucose, du dextrose, du fructose, du man-nitol, du xylitol, du sorbitol, de l'isomalt, du sirop de maltitol, du lactitol, du leucrose, des fructooligosaccharides, des glucanes, du polyglucose et/ou des mélanges de ces substances.

11. Composition à utiliser selon l'une quelconque des revendications précédentes, dans laquelle la composition comprend au moins un support liquide,
en particulier dans laquelle le support comprend des solvants polaires et/ou apolaires, de préférence de l'eau, de l'éthanol, du glycérol, de l'huile de ricin hydrogénée, du polyéthylène glycol, du polypropylène glycol et/ou des mélanges de ceux-ci.

12. Composition à utiliser selon l'une quelconque des revendications précédentes, dans laquelle la composition contient au moins un autre ingrédient,
en particulier, dans laquelle l'au moins un autre ingrédient est choisi dans le groupe des additifs et/ou des adjuvants, de préférence des agents liants, mouillants, stabilisants, colorants, tampons, olfactifs, aromatiques, édulcorants, aromatisants, des auxiliaires de fabrication et des agents de conservation et/ou de leurs mélanges.

13. Composition à utiliser selon l'une des revendications précédentes,
dans laquelle la composition est préparée pour la consommation et/ou pour une application topique, en particulier dans la cavité buccale; et/ou
dans laquelle la composition est préparée sous forme solide, liquide, visqueuse (semblable à un gel), semi-solide, en aérosol ou pâteuse, en particulier sous forme liquide ou solide; et/ou
dans laquelle la composition est préparée sous forme de boisson, de barre, de pâtisserie, de caramel dur, de chewing-gum, de dentifrice et/ou de bain de bouche, en particulier sous forme de boisson, de caramel dur, de chewing-gum ou de bain de bouche, de préférence sous forme de boisson.
